# EUROPEAN PATENT SPECIFICATION

(11) **EP 1 097 210 B1**
(45) Date of publication and mention of the grant of the patent: **14.06.2006**
(21) Application number: 99932859.4
(22) Date of filing: 09.07.1999
(51) Int. Cl.: C12N 15/13, C12N 15/70, C12N 15/85, C12N 1/21, C12N 5/10, C07K 16/28, C07K 16/46, A61K 31/70, A61K 39/395, G01N 33/577, G01N 33/68, C12Q 1/68, A01K 67/027

(54) **IMMUNOLOGICAL REAGENT SPECIFICALLY INTERACTING WITH THE EXTRACELLULAR DOMAIN OF THE HUMAN ZETA CHAIN**
IMMUNOLOGISCHES REAGENS, DAS SPEZIFISCH MIT DER EXTRAZELLULÄREN DOMÄNE DER MENSCHLICHEN ZETA-KETTE REAGIERT
REACTIF IMMUNOLOGIQUE CAPABLE D'INTERAGIR SPECIFIQUEMENT AVEC LE DOMAINE EXTRACELLULAIRE DE LA CHAINE ZETA HUMAINE

(30) Priority: 10.07.1998 EP 98112867
(43) Date of publication of application: 09.05.2001
(73) Proprietor: Connex GmbH, 82152 Martinsried (DE)
(72) Inventor: REITER, Christian, D-85757 Karlsfeld (DE)
(74) Representative: Vossius & Partner
(86) International application number: PCT/EP1999/004838
(87) International publication number: WO 2000/003016

(56) References cited:
- WO-A-90/15822
- M. MACK ET AL.: "Biologic properties of a bispecific single-chain antibody directed against 17-1A (EpCAM) and CD3: tumor cell-dependent T cell stimulation and cytotoxic activity." THE JOURNAL OF IMMUNOLOGY, vol. 158, no. 8, 15 April 1997 (1997-04-15), pages 3965-3970, XP002100040 Baltimore, MD, USA cited in the application
- A. TRAUNECKER ET AL.: "Bispecific single chain molecules (Janusins) target cytotoxic lymphocytes on HIV infected cells." THE EMBO JOURNAL, vol. 10, no. 12, December 1991 (1991-12), pages 3655-3659, XP000232579 Oxford, GB
- W. HELFRICH ET AL.: "Construction and characterization of a bispecific diabody for retargeting T cells to human carcinomas." INTERNATIONAL JOURNAL OF CANCER, vol. 76, no. 2, 13 April 1998 (1998-04-13), pages 232-239, XP002121156 Copenhagen, Denmark
- C. RENNER ET AL.: "T cells from patients with Hodgkin's disease have a defective T-cell receptor zeta chain expression that is reversible by T-cell stimulation with CD3 and CD28." BLOOD, vol. 88, no. 1, 1 July 1996 (1996-07-01), pages 236-241, XP002121157 New York, NY, USA
- J. SMITH ET AL.: "Nonmitogenic anti-CD3 monoclonal antibodies deliver a partial T cell recetor signal and induce clonal anergy." THE JOURNAL OF EXPERIMENTAL MEDICINE, vol. 185, no. 8, 21 April 1997 (1997-04-21), pages 1413-1422, XP002121158 New York, NY, USA

### SEQUENCE LISTING

<110> Connex GmbH
<120> Immunological reagent specifically interacting with the extracellular domain of the human zeta chain
<130> C1368PCT
<140>
   <141>
<150> EP 98 11 2867.1
   <151> 1998-07-10
<160> 18
<170> PatentIn Ver. 2.1
<210> 1
   <211> 33
   <212> DNA
   <213> Rattus norvegicus
<220>
   <221> CDS
   <222> (1) .. (33)
<400> 1
   cag gca agc cag gac att ggt aat tgg tta gca                         33
   Gln Ala Ser Gln Asp Ile Gly Asn Trp Leu Ala
     1               5                  10
<210> 2
   <211> 11
   <212> PRT
   <213> Rattus norvegicus
<400> 2
   Gln Ala Ser Gln Asp Ile Gly Asn Trp Leu Ala
     1               5                  10
<210> 3
   <211> 21
   <212> DNA
   <213> Rattus norvegicus
<220>
   <221> CDS
   <222> (1)..(21)
<400> 3
   agt gca acc agc ttg gca gac                                         21
   Ser Ala Thr Ser Leu Ala Asp
     1               5
<210> 4
   <211> 7
   <212> PRT
   <213> Rattus norvegicus
<400> 4
   Ser Ala Thr Ser Leu Ala Asp
   1 5
<210> 5
   <211> 27
   <212> DNA
   <213> Rattus norvegicus
<220>
   <221> CDS
   <222> (1)..(27)
<400> 5
   cta cag cgt tat agt aat ccc aac acg                          27
   Leu Gln Arg Tyr Ser Asn Pro Asn Thr
   1               5
<210> 6
   <211> 9
   <212> PRT
   <213> Rattus norvegicus
<400> 6
   Leu Gln Arg Tyr Ser Asn Pro Asn Thr
   1               5
<210> 7
   <211> 30
   <212> DNA
   <213> Rattus norvegicus
<220>
   <221> CDS
   <222> (1)..(30)
<400> 7
   ggc tac aca ttc acc agt tac gat atg cac                      30
   Gly Tyr Thr Phe Thr Ser Tyr Asp Met His
   1               5                   10
<210> 8
   <211> 10
   <212> PRT
   <213> Rattus norvegicus
<400> 8
   Gly Tyr Thr Phe Thr Ser Tyr Asp Met His
   1               5                   10
<210> 9
   <211> 51
   <212> DNA
   <213> Rattus norvegicus
<220>
   <221> CDS
   <222> (1)..(51)
<400> 9
   tgg att tat cct gga aat ggt aat act aag tac aat caa aag ttc aat      48
   Trp Ile Tyr Pro Gly Asn Gly Asn Thr Lys Tyr Asn Gln Lys Phe Asn
    1               5                  10                  15
   ggg                                                                   51
   Gly
<210> 10
   <211> 17
   <212> PRT
   <213> Rattus norvegicus
<400> 10
   Trp Ile Tyr Pro Gly Asn Gly Asn Thr Lys Tyr Asn Gln Lys Phe Asn
    1               5                  10                  15
   Gly
<210> 11
   <211> 42
   <212> DNA
   <213> Rattus norvegicus
<220>
   <221> CDS
   <222> (1)..(42)
<400> 11
   gat tgg cat tac tat agc agc tat atc cgt ccc ttt gct tac              42
   Asp Trp His Tyr Tyr Ser Ser Tyr Ile Arg Pro Phe Ala Tyr
    1               5                  10
<210> 12
   <211> 14
   <212> PRT
   <213> Rattus norvegicus
<400> 12
   Asp Trp His Tyr Tyr Ser Ser Tyr Ile Arg Pro Phe Ala Tyr
   1 5 10
<210> 13
   <211> 369
   <212> DNA
   <213> Rattus norvegicus
<220>
   <221> CDS
   <222> (1)..(369)
<400> 13
   cag gta cag ctg cag caa tct ggg gct gaa cta gtg aag cct ggg tcc     48
   Gln Val Gln Leu Gln Gln Ser Gly Ala Glu Leu Val Lys Pro Gly Ser
    1               5                  10                  15
   tca gtg aaa att tcc tgc aag gct tct ggc tac aca ttc acc agt tac     96
   Ser Val Lys Ile Ser Cys Lys Ala Ser Gly Tyr Thr Phe Thr Ser Tyr
               20                  25                  30
   gat atg cac tgg ata aaa cag cag cct gga aat ggc ctt gag tgg att    144
   Asp Met His Trp Ile Lys Gln Gln Pro Gly Asn Gly Leu Glu Trp Ile
           35                  40                  45
   ggg tgg att tat cct gga aat ggt aat act aag tac aat caa aag ttc    192
   Gly Trp Ile Tyr Pro Gly Asn Gly Asn Thr Lys Tyr Asn Gln Lys Phe
       50                  55                  60
   aat ggg aag gca aca ctc act gca gac aaa tcc tcc agc aca gcc tat    240
   Asn Gly Lys Ala Thr Leu Thr Ala Asp Lys Ser Ser Ser Thr Ala Tyr
   65                  70                  75                  80
   atg cag ctc agc agc ctg aca tct gag gac tct gca gtc tat ttc tgt    288
   Met Gln Leu Ser Ser Leu Thr Ser Glu Asp Ser Ala Val Tyr Phe Cys
                   85                  90                  95
   gca aga gat tgg cat tac tat agc agc tat atc cgt ccc ttt gct tac    336
   Ala Arg Asp Trp His Tyr Tyr Ser Ser Tyr Ile Arg Pro Phe Ala Tyr
               100                 105                 110
   tgg ggc caa ggc act ctg gtc act gtc tct tca                        369
   Trp Gly Gln Gly Thr Leu Val Thr Val Ser Ser
           115                 120
<210> 14
   <211> 123
   <212> PRT
   <213> Rattus norvegicus
<400> 14
   Gln Val Gln Leu Gln Gln Ser Gly Ala Glu Leu Val Lys Pro Gly Ser
   1               5                   10                  15
   Ser Val Lys Ile Ser Cys Lys Ala Ser Gly Tyr Thr Phe Thr Ser Tyr
               20                  25                  30
   Asp Met His Trp Ile Lys Gln Gln Pro Gly Asn Gly Leu Glu Trp Ile
           35                  40                  45
   Gly Trp Ile Tyr Pro Gly Asn Gly Asn Thr Lys Tyr Asn Gln Lys Phe
       50                  55                  60
   Asn Gly Lys Ala Thr Leu Thr Ala Asp Lys Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Arg Asp Trp His Tyr Tyr Ser Ser Tyr Ile Arg Pro Phe Ala Tyr
               100                 105                 110
   Trp Gly Gln Gly Thr Leu Val Thr Val Ser Ser
           115                 120
<210> 15
   <211> 321
   <212> DNA
   <213> Rattus norvegicus
<220>
   <221> CDS
   <222> (1)..(321)
<400> 15
   gac atc cag atg aca cag tct cct gct tcc ctg tct gcg tct ccg gaa    48
   Asp Ile Gln Met Thr Gln Ser Pro Ala Ser Leu Ser Ala Ser Pro Glu
     1               5                  10                  15
   gaa att gtc acg atc aca tgc cag gca agc cag gac att ggt aat tgg    96
   Glu Ile Val Thr Ile Thr Cys Gln Ala Ser Gln Asp Ile Gly Asn Trp
                20                  25                  30
   tta gca tgg tat cag cag aaa cca ggg aaa tct cct caa ctc ctg atc   144
   Leu Ala Trp Tyr Gln Gln Lys Pro Gly Lys Ser Pro Gln Leu Leu Ile
            35                  40                  45
   tat agt gca acc agc ttg gca gac ggg atc cca tca agg ttc agc ggc   192
   Tyr Ser Ala Thr Ser Leu Ala Asp Gly Ile Pro Ser Arg Phe Ser Gly
       50                  55                  60
   agt aga tct ggt aca cag tat tct ctt aag atc agc aga cta cag gtt   240
   Ser Arg Ser Gly Thr Gln Tyr Ser Leu Lys Ile Ser Arg Leu Gln Val
    65                  70                  75                  80
   gaa gat act gga atc tat tac tgt cta cag cgt tat agt aat ccc aac   288
   Glu Asp Thr Gly Ile Tyr Tyr Cys Leu Gln Arg Tyr Ser Asn Pro Asn
                    85                  90                  95
   acg ttt gga gct ggg acc aag ctg gag ctg aaa                       321
   Thr Phe Gly Ala Gly Thr Lys Leu Glu Leu Lys
               100                 105
<210> 16
   <211> 107
   <212> PRT
   <213> Rattus norvegicus
<400> 16
   Asp Ile Gln Met Thr Gln Ser Pro Ala Ser Leu Ser Ala Ser Pro Glu
     1               5                  10                  15
   Glu Ile Val Thr Ile Thr Cys Gln Ala Ser Gln Asp Ile Gly Asn Trp
                20                  25                  30
   Leu Ala Trp Tyr Gln Gln Lys Pro Gly Lys Ser Pro Gln Leu Leu Ile
            35                  40                  45
   Tyr Ser Ala Thr Ser Leu Ala Asp Gly Ile Pro Ser Arg Phe Ser Gly
       50                  55                  60
   Ser Arg Ser Gly Thr Gln Tyr Ser Leu Lys Ile Ser Arg Leu Gln Val
    65                  70                  75                  80
   Glu Asp Thr Gly Ile Tyr Tyr Cys Leu Gln Arg Tyr Ser Asn Pro Asn
                    85                  90                  95
   Thr Phe Gly Ala Gly Thr Lys Leu Glu Leu Lys
<210> 17
   <211> 1637
   <212> DNA
   <213> Artificial Sequence
<220>
   <223> Description of Artificial Sequence: artificial sequence
<400> 17
   gaattcacca tgggatggag ctgtatcatc ctcttcttgg tagcaacagc tacaggtgta 60
   cactccgata tccagatgac acagtctcct gcttccctgt ctgcgtcccc ggaagaaatt 120
   gtcacgatca catgccaggc aagccaggac attggtaatt ggttagcatg gtatcagcag 180
   aaaccaggga aatctcctca actcctgatc tatagtgcaa ccagcttggc agacgggatc 240
   ccatcaaggt tcagcggcag tagatctggt acacagtatt ctcttaagat cagcagacta 300
   caggttgaag atactggaat ctattactgt ctacagcgtt atagtaatcc caacacgttt 360
   ggagctggga ccaagctgga gctgaaaggt ggtggtggtt ctggcggcgg cggctccggt 420
   ggtggtggtt ctcaggtaca gctgcagcaa tctggagctg agctagtgaa gcctgggtcc 480
   tcagtgaaaa tttcctgcaa ggcttctggc tacacattca ccagttacga tatgcactgg 540
   ataaaacagc agcctggaaa tggccttgag tggattgggt ggatttatcc tggaaatggt 600
   aatactaagt acaatcaaaa gttcaatggg aaggcaacac tcactgcaga caaatcctcc 660
   agcacagcct atatgcagct cagcagcctg acatctgagg actctgcagt ctatttctgt 720
   gcaagagatt ggcattacta tagcagctat atccgtccct ttgcttactg gggccaaggc 780
   actctggtca ctgtctcttc cggaggtggt ggttctgagg tgcagctgct cgagcagtct 840
   ggagctgagc tggcgaggcc tggggcttca gtgaagctgt cctgcaaggc ttctggctac 900
   accttcacaa actatggttt aagctgggtg aagcagaggc ctggacaggt ccttgagtgg 960
   attggagagg tttatcctag aattggtaat gcttactaca atgagaagtt caagggcaag 1020
   gccacactga ctgcagacaa atcctccagc acagcgtcca tggagctccg cagcctgacc 1080
   tctgaggact ctgcggtcta tttctgtgca agacggggat cctacgatac taactacgac 1140
   tggtacttcg atgtctgggg ccaagggacc acggtcaccg tctcctcagg tggtggtggt 1200
   tctggcggcg gcggctccgg tggtggtggt tctgagctcg tgatgaccca gactccactc 1260
   tccctgcctg tcagtcttgg agatcaagcc tccatctctt gcagatctag tcagagcctt 1320
   gtacacagta atggaaacac ctatttacat tggtacctgc agaagccagg ccagtctcca 1380
   aagctcctga tctacaaagt ttccaaccga ttttctgggg tcccagacag gttcagtggc 1440
   agtggatcag ggacagattt cacactcaag atcagcagag tggaggctga ggatctggga 1500
   gtttatttct gctctcaaag tacacatgtt ccgtacacgt tcggaggggg gaccaagctt 1560
   gagatcaaac gtacgactag ccatcaccat caccatcaca ctagctaatt aatttaagcg 1620
   gccgctctag agtcgac                                                1637
<210> 18
   <211> 532
   <212> PRT
   <213> Artificial Sequence
<220>
   <223> Description of Artificial Sequence: artificial sequence
<400> 18
   Met Gly Trp Ser Cys Ile Ile Leu Phe Leu Val Ala Thr Ala Thr Gly
   1               5                   10                  15
   Val His Ser Asp Ile Gln Met Thr Gln Ser Pro Ala Ser Leu Ser Ala
               20                  25                  30
   Ser Pro Glu Glu Ile Val Thr Ile Thr Cys Gln Ala Ser Gln Asp Ile
           35                  40                  45
   Gly Asn Trp Leu Ala Trp Tyr Gln Gln Lys Pro Gly Lys Ser Pro Gln
   50 55 60
   Leu Leu Ile Tyr Ser Ala Thr Ser Leu Ala Asp Gly Ile Pro Ser Arg
   65 70 75 80
   Phe Ser Gly Ser Arg Ser Gly Thr Gln Tyr Ser Leu Lys Ile Ser Arg
   85 90 95
   Leu Gln Val Glu Asp Thr Gly Ile Tyr Tyr Cys Leu Gln Arg Tyr Ser
   100 105 110
   Asn Pro Asn Thr Phe Gly Ala Gly Thr Lys Leu Glu Leu Lys Gly Gly
   115 120 125
   Gly Gly Ser Gly Gly Gly Gly Ser Gly Gly Gly Gly Ser Gln Val Gln
   130 135 140
   Leu Gln Gln Ser Gly Ala Glu Leu Val Lys Pro Gly Ser Ser Val Lys
   145 150 155 160
   Ile Ser Cys Lys Ala Ser Gly Tyr Thr Phe Thr Ser Tyr Asp Met His
   165 170 175
   Trp Ile Lys Gln Gln Pro Gly Asn Gly Leu Glu Trp Ile Gly Trp Ile
   180 185 190
   Tyr Pro Gly Asn Gly Asn Thr Lys Tyr Asn Gln Lys Phe Asn Gly Lys
   195 200 205
   Ala Thr Leu Thr Ala Asp Lys Ser Ser Ser Thr Ala Tyr Met Gln Leu
   210 215 220
   Ser Ser Leu Thr Ser Glu Asp Ser Ala Val Tyr Phe Cys Ala Arg Asp
   225 230 235 240
   Trp His Tyr Tyr Ser Ser Tyr Ile Arg Pro Phe Ala Tyr Trp Gly Gln
   245 250 255
   Gly Thr Leu Val Thr Val Ser Ser Gly Gly Gly Gly Ser Glu Val Gln
   260 265 270
   Leu Leu Glu Gln Ser Gly Ala Glu Leu Ala Arg Pro Gly Ala Ser Val
   275 280 285
   Lys Leu Ser Cys Lys Ala Ser Gly Tyr Thr Phe Thr Asn Tyr Gly Leu
   290 295 300
   Ser Trp Val Lys Gln Arg Pro Gly Gln Val Leu Glu Trp Ile Gly Glu
   305 310 315 320
   Val Tyr Pro Arg Ile Gly Asn Ala Tyr Tyr Asn Glu Lys Phe Lys Gly
   325 330 335
   Lys Ala Thr Leu Thr Ala Asp Lys Ser Ser Ser Thr Ala Ser Met Glu
   340 345 350
   Leu Arg Ser Leu Thr Ser Glu Asp Ser Ala Val Tyr Phe Cys Ala Arg
   355 360 365
   Arg Gly Ser Tyr Asp Thr Asn Tyr Asp Trp Tyr Phe Asp Val Trp Gly
   370 375 380
   Gln Gly Thr Thr Val Thr Val Ser Ser Gly Gly Gly Gly Ser Gly Gly
   385 390 395 400
   Gly Gly Ser Gly Gly Gly Gly Ser Glu Leu Val Met Thr Gln Thr Pro
                   405                 410                 415
   Leu Ser Leu Pro Val Ser Leu Gly Asp Gln Ala Ser Ile Ser Cys Arg
               420                 425                 430
   Ser Ser Gln Ser Leu Val His Ser Asn Gly Asn Thr Tyr Leu His Trp
           435                 440                 445
   Tyr Leu Gln Lys Pro Gly Gln Ser Pro Lys Leu Leu Ile Tyr Lys Val
       450                 455                 460
   Ser Asn Arg Phe Ser Gly Val Pro Asp Arg Phe Ser Gly Ser Gly Ser
   465                 470                 475                 480
   Gly Thr Asp Phe Thr Leu Lys Ile Ser Arg Val Glu Ala Glu Asp Leu
                   485                 490                 495
   Gly Val Tyr Phe Cys Ser Gln Ser Thr His Val Pro Tyr Thr Phe Gly
               500                 505                 510
   Gly Gly Thr Lys Leu Glu Ile Lys Arg Thr Thr Ser His His His His
           515                 520                 525
   His His Thr Ser
       530

## Description

The present invention relates to a nucleic acid molecule comprising a nucleic acid sequence encoding three complementary determining regions (CDRs) of a variable region of an antibody, said antibody specifically interacting with the extracellular domain of the human zeta-chain on the surface of intact cells, said antibody being obtainable by immunizing a rat with Jurkat cells and subsequently with a conjugate comprising a carrier molecule and a peptide comprising the 11 N-terminal amino acids of the rat zeta-chain wherein said 11 N-terminal amino acids consist of the sequence QSFGLLDPKLC, wherein said nucleic acid sequence encodes a V_{H} chain or wherein said nucleic acid sequence encodes a V_{L} chain. Preferably, the (poly)peptide encoded by the nucleic acid molecule of the invention is a monospecific or bispecific antibody. The invention also relates to pharmaceutical compositions comprising i. a. the nucleic acid molecule or antibody of the invention as well as to kits comprising the aforementioned compounds. Finally, the invention relates to an in vitro method for the determination of zeta-chain or eta-chain expression on NK-cells, T-cells or precursors thereof employing the antibody of the invention.

The zeta-chain is part of a family of structurally and functionally related signal transduction molecules, further encompassing the eta-chain (an alternatively spliced form of the zeta-chain) and the gamma-chain of the high affinity IgE-Fc-receptor FcεRI. Common traits within this family of transmembrane proteins are their long intracellular domain comprising one or several ITAM sequence motifs (immunoreceptor tyrosine-based activation motif; zeta: 3, eta: 2, gamma: 1) as well as extremely short extracellular domains, constituted of 9 (zeta, eta, sequence identical) or 4 (FcεRI-gamma) amino acids. The sequence of the extracellular domains of these proteins is 100% conserved between mouse, rat and man, and most likely other species as well.
The zeta-chain is expressed as a homodimer or as a heterodimer with the eta-chain on T-lymphocytes, natural killer- (NK-) cells and, to some extent, their precursors, exclusively. On the surface of mature T-lymphocytes the zeta chain is structurally and functionally closely associated with the T-cell receptor (TCR) and the CD3-complex. Signals induced through engagement of the TCR are transduced into the cytoplasm of the T-cell via CD3 and the zeta-chain, with the three ITAMs of the zeta-chain providing the major part of the signal amplification effect compared to the single ITAM on the epsilon-, delta- and gamma-chain (different from FcεRI-gamma) that constitute the CD3-complex.
On the surface of NK-cells, the zeta-chain shows a similar association with the IgG-Fc-receptor (FcγRIIIA). When antibody-coated target cells are recognized by NK-cells via FcγRIIIA, the resulting signal is transduced to the cytoplasm through the zeta- and/or the gamma-chain, thus activating the NK-cell which consecutively lyses the target cell that was recognized (ADCC, antibody dependent cellular cytotoxicity).

The TCR-complex on mature T-lymphocytes is thus an oligomeric structure, composed of multiple chains (TCR-α/β or TCR-γ/δ associated with CD3ε, CD3δ, CD3γ and the zeta chain or its alternative splice-product eta) (Keegan, Immunology Today 13 (1992) 63-68). Antigen recognition is accomplished by the polymorphic TCR-α/β- or TCR-γ/δ-heterodimers that are devoid of intracytoplasmatic signal transduction domains. The invariant CD3 proteins (γ/ε- and δ/ε-heterodimers) and the zeta or eta chain (zeta-homodimers or zeta-eta-heterodimers) are necessary for correct assembly, transport and efficient cell surface expression of the whole TCR-complex and transduce TCR signals (Clevers, Annu. Rev. Immunol. 6 (1988) 629-662, Ashwell, Annu. Rev. Immunol. 8 (1990) 139-167). Signaling requires a conserved 18-amino acid sequence (Reth, Nature 338 (1989) 383-384, Samelson, J. Biol. Chem. 267 (1992) 24913-24916), termed the immunoreceptor tyrosine-based activation motif (ITAM), which is found three times in the zeta chain, twice in the eta chain and once in each of the CD3 subunits (γ, δ and ε). Each ITAM contains a pair of tyrosine-X-X-leucine/isoleucine (Y-X-X-L/I) motifs, that are separated by 10 or 11 amino acids (Cambier, Immunology Today 16 (1995) 110). The tyrosine residues in each ITAM are rapidly phosphorylated after TCR ligation and serve as docking sites for signaling proteins that can bind to the phosphotyrosine residues by src-homology-2 (SH2) domains (Cooke, Cell 65 (1991) 281-291, Glaischenhaus, Cell 64 (1991) 511-520, Samelson, Proc. Natl. Acad. Sci. USA. 87 (1990) 4358-4362, Straus, Cell 70 (1992) 585-593, Songyang, Cell 72 (1993) 767-778, Songyang, Mol. Cell. Biol. 14 (1994) 2777-2785, Isakov, J. Exp. Med. 181 (1995) 375-380). The necessity of the zeta-chain in TCR-mediated signaling was demonstrated by studies of a zeta-deficient mutant derivative of an antigen-specific T cell hybridoma; the mutant line was incapable of responding to antigen and only poorly responsive to anti-CD3 antibodies (Sussman, Cell 52 (1988) 85-95). Although the retention of some activity in response to anti-CD3 antibody stimulation suggested the other chains of the TCR-complex were able to compensate for the absence of zeta, studies in which the mutant line was shown to reacquire the ability to recognize antigen or to respond to anti-CD3 antibodies when reconstituted with zeta-chain by transfection clearly document an important role for the zeta-chain in signal transduction (Weissman, EMBO J. 8 (1989) 3651-3656). Due to its singular configuration, the zeta chain may function as the predominant TCR signaling structure and its triplicated ITAMs may serve primarily to facilitate TCR signal amplification.
The TCR-complex in general and the zeta-chain mediated signal transduction in particular are involved in both the activation and the programmed cell death (apoptosis) of mature T-lymphocytes. Experiments in which the zeta-chain cytoplasmatic tail was attached to unrelated receptors showed that cell lines expressing these chimeric receptors could respond to antibody crosslinking with IL-2 release and upregulation of other activation parameters (Irving, Cell 64 (1991) 891-901). Furthermore, cytotoxic T lymphocytes (CTL) expressing chimeric zeta-chain derivatives were shown to specifically lyse target cells bearing surface molecules recognized by the chimeric zeta-receptor (Romeo, Cell 64 (1991) 1037-1046, Romeo, Cell 68 (1992) 889-897). This, however, proved to be true only in case of activated T cells since resting T lymphocytes expressing chimeric zeta-chain molecules could neither be activated nor show any cytotoxicity against target cells when stimulated through these chimeric receptors (Brocker, J. Exp. Med. 181 (1995) 1653-1659), that according to biochemical analyses do not associate with endogenous TCR-subunits and therefore act as physically independent signaling molecules (Shinkai, Immunity 2 (1995) 401-411). These data thus indicate that chimeric zeta-chain derivatives can only substitute for the complete TCR-complex in activated but not in resting T cells. TCR-mediated apoptosis of mature T lymphocytes may be induced if strong TCR reengagement occurs when the cells are activated and proliferating (Lenardo, Nature 353 (1991) 858-861, Russell, Proc. Natl. Acad. Sci. USA. 88 (1991) 2151-2157, Critchfield, Cell. Immunol. 160 (1995) 71-78). Mature T cell death plays a critical role in peripheral immune homeostasis and tolerance. Recent experiments show that the zeta-chain is required for efficient induction of T-cell apoptosis through engagement of the TCR and that the signaling domains of CD3 only have a minor (CD3ε) or no (CD3γ and δ) effect on TCR-mediated apoptosis (Combadière, J. Exp. Med. 183 (1996) 2109-2117). In addition, the three zeta-chain ITAMs contribute differently to the induction of T-cell apoptosis, with the most N-terminal one having the predominant effect followed by the C-terminal ITAM resembling the low contribution of CD3ε and the middle one being completely incapable of inducing apoptosis.

T cell development takes place primarily in the thymus, where T cell precursors immigrate from the fetal liver or from adult bone marrow. Upon immigration into the thymus these early progenitor T cells are triple negative (TN: TCR⁻ CD4⁻ CD8⁻) (Shortman, Annu. Rev. Immunol. 14 (1996) 29-47) but already express the zeta chain and CD3 chains (Wiest, J. Exp. Med. 180 (1994) 1375-1382, Wilson, Int. Immunol. 7 (1995) 1659-1664). In the inductive environment of the thymus they transit a series of developmental stages prior to their differentiation into CD4⁺CD8⁺ double positive (DP) thymocytes (Godfrey, Immunol. Today 14 (1993) 547-553). The most immature CD44⁺CD25⁻-TN-thymocytes and CD44⁺CD25⁺-TN-thymocytes derived therefrom still show germline configuration of the TCR-genes. TN-thymocytes of the next maturation stage characterized by the surface phenotype CD44^{-/lo}CD25⁺, however, start to rearrange the TCRβ locus. Up to this stage the zeta chain, although expressed, is probably not required for thymocyte maturation (Crompton, Eur. J. Immunol. 24 (1994) 1903-1907). The following maturation step of TN-thymocytes is characterized by the phenotype switch from CD44^{-/lo}CD25⁺ to CD44⁻CD25⁻, however, it is blocked in the absence of the zeta-chain (Crompton, Eur. J. Immunol. 24 (1994) 1903-1907) and requires rearrangement and expression of the TCRβ chain (Kishi, EMBO J. 10 (1991) 93-100, Mombaerts, Nature 360 (1992) 225-231, Shinkai, Science 259 (1993) 822-825). The TCRβ chain associates with an invariant chain termed pre-Tα (pTα) (Groettrup, Cell 75 (1993) 283-294), that substitutes for the still unrearranged TCRα chain to form the pre-TCR probably associated with the zeta-chain and CD3-chains (Van Oers, J. Exp. Med. 182 (1995) 1585-1590). As a consequence of signals mediated by the pre-TCR, thymocytes progress in development to the DP stage. At this stage, thymocytes initiate rearrangement of their TCRα genes, cease to express pTα, and start to express low levels of TCR complexes, resembling the multiple chain composition of the TCR-complex on mature T-lymphocytes (Von Boehmer, Ann. N. Y. Acad. Sci. 766 (1995) 52-61, Robey, Annu. Rev. Immunol. 12 (1994) 265-705).
Since the development of CD44⁻CD25⁻-TN thymocytes and subsequently that of DP thymocytes is inhibited in the absence of the zeta-chain and can be restored by the expression of a signaling deficient mutant zeta-chain without functional ITAMs, respectively (Shores, J Immunol 159 (1997) 222-230, Shores, Science 266 (1994) 1047-1050), the importance of the zeta-chain related to the promotion of the pre-TCR surface expression may exceed that related to its signaling potential.
DP thymocytes are further subjected to selection on the basis of the specificity of their TCRs. Thymocytes expressing TCRs with negligible specificity for self-MHC molecules, irrespective of which peptide is bound by the MHC-protein, die within the thymus, presumably because they fail to receive survival signals through their TCR (Robey, Annu. Rev. Immunol. 12 (1994) 265-705, Jameson, Annu. Rev. Immunol. 13 (1995) 93-126). In contrast, thymocytes that express TCRs with the appropriate ligand specificities survive and mature to either CD4⁺- or CD8⁺-single positive (SP) T-cells showing high level TCR-expression. However, thymocytes expressing autoreactive or potentially autoreactive TCRs are deleted (Robey, Annu. Rev. Immunol. 12 (1994) 265-705, Jameson, Annu. Rev. Immunol. 13 (1995) 93-126). Thus, engagement of the TCR on DP thymocytes leads to two dramatically different cell fates, either survival and further maturation (positive selection) or death by apoptosis (negative selection).
Although the zeta-chain ITAMs are not essential for the development (Shores, Science 266 (1994) 1047-1050) and MHC-restricted selection (Simpson, Int. Immunol. 7 (1995) 287-293) of mature SP T-cells in the thymus, they seem to contribute to the shaping of the T-cell repertoire by amplifying the signaling response generated by TCR-engagement during thymocyte selection.
Indeed, the results of a recent study revealed that, although no individual ITAM was specifically required, there was a direct relationship between the number of zeta-chain ITAMs within the TCR complex and the efficiency of both positive and negative selection (Shores, J. Exp. Med. 185 (1997) 893-900). These results might be expected if positive and negative selection are dictated primarily by TCR signaling thresholds and if the magnitude of the signaling response to ligands of different affinity, critical in determining the fate of developing thymocytes, is more or less amplified by the triplicated ITAMs of the native zeta-chain or by a reduced number of ITAMs in zeta-chain mutants, respectively.
Thus it is expected, that the repertoire of positively selected thymocytes in the presence of the native zeta-chain markedly differs from that selected in the presence of a signaling deficient zeta chain derivative, and that the latter repertoire contains T-cells that might otherwise be negatively selected (Shores, Current Opinion in Immunology 9 (1997) 380-389).

NK cells are large granular lymphocytes that make up 10 to 15% of peripheral blood lymphocytes (PBL). They are capable of killing tumor cells and certain virally infected cells in a manner not restricted by the major histocompatibility complex (MHC) (Trinchieri, Adv. Immunol. 47 (1989) 187-376, Ritz, Adv. Immunol. (1988) 181-211). This cytolytic effector function does not require prior sensitization or antigen presentation by accessory cells. These properties allow NK-cells to effect an innate host defense prior to the elicitation of an antigen-specific immune response. NK-cells are known to effect two forms of cytolytic activity, natural cytotoxicity and ADCC (antibody dependent cellular cytotoxicity) that also kills target cells resistant to natural cytotoxicity.
Unlike the cytotoxic activity of T-cells that is triggered by an activation signal generated through engagement of the clonotypic TCR, natural cytotoxicity of NK-cells is primarily regulated by inhibitory signals (Yokoyama, J. Exp. Med. 186 (1997) 1803-1808). The engagement of inhibitory NK-cell receptors by specific binding to MHC class I molecules on target cells, leads to inhibition of natural cytotoxicity, that is released in the absence of target cell MHC class I expression, thus allowing the activation of natural killing. NK-cell receptors contain intracytoplasmatic immunoreceptor tyrosine-based inhibitory motifs (ITIM, consensus sequence I/V-X-Y-X-X-L) that mediate inhibitory signals after tyrosine phosphorylation induced by receptor engagement (Muta, Nature 368 (1994) 70-73, Thomas, J. Exp. Med. 181 (1995) 1953-1956).

ADCC is mediated by the low affinity IgG Fc-receptor FcγRIIIA and can be demonstrated in vitro by incubating NK-cells with antibody-coated target cells. Ligand binding and crosslinking of FcγRIIIA induce NK-cell activation resulting in cytolytic activity, up-regulation of surface activation molecules and cytokine secretion (Chehimi, J. Exp. Med. 75 (1992) 789, Ravetch, Annu. Rev. Immunol. 9 (1991) 457). FcγRIIIA is expressed as a complex comprising the transmembrane ligand-binding receptor glycoprotein CD16 and two membrane-spanning chains, gamma and zeta, which are responsible for both receptor assembly and signal transduction (Anderson, Proc. Natl. Acad. Sci. USA. 87 (1990) 2274-2278, Ravetch, Annu. Rev. Immunol. 9 (1991) 457). The gamma chain was originally identified as a subunit of the high affinity receptor for IgE and belongs to the same family of signal transduction molecules together with the zeta- and the eta-chain. Upon engagement of FcγRIIIA on NK-cells tyrosine phosphorylation occurs within the ITAMs of the zeta- and gamma-chain thus inducing further signal transduction events including the recruitment of specific Src homology (SH)2 domain containing proteins to the FcγRIIIA-complex. As a recent study demonstrated, the FcγRIIIA-mediated NK-cell activation seems to be imitated by the engagement of chimeric zeta-chain receptors transfected into NK-cells, thus resembling analogous approaches in T-cells (Tran, J. Immunol. 155 (1995) 1000-1009).

Furthermore, the prior art discloses the production of a single chain bispecific antibody directed to CD3 and EpCAM to be used to redirect cytotoxic T cells to tumor cells in an MHC-unrestricted fashion (M. Mack et al., The Journal of Immunology, (1997), 3965 - 3970).

The construction of a recombinant bispecific antibody fragment in the diabody format, specific for both the CD3 ∈-chain and for the tumor antigen EGP2 is also disclosed in the prior art. This diabody is useful to retarget activated cytotoxic T cell to lyse various human carcinoma in vivo (W. Helfrich et a., International Journal of Cancer, (1998), 232 - 239).

Whereas it becomes apparent from the above that antibodies specifically interacting with /recognizing the extracellular domain of the human zeta chain on the surface of intact cells were in great demand for a variety of purposes such as artificial signal transduction on T cells or NK-cells, for example, in the treatment of tumors, to date no successful experiments have been reported. The lack of success in producing an antibody that fulfills this need may primarily be due to the rather short length (9 amino acids) of this domain, possibly in conjunction with the association of the zeta-chain with the T-cell receptor and the CD3 complex on T-cells or with the IgG-Fc-receptor on NK-cells.

The technical problem underlying the present invention was therefore to provide a tool that may successfully be applied to the above-identified need. The solution to this technical problem is achieved by providing the embodiments characterized in the claims.

Accordingly, the present invention relates to a nucleic acid molecule comprising a nucleic acid sequence encoding three complementary determining regions (CDRs) of a variable region of an antibody, said antibody specifically interacting with the extracellular domain of the human zeta-chain on the surface of intact cells, said antibody being obtainable by immunizing a rat with Jurkat cells and subsequently with a conjugate comprising a carrier molecule and a peptide comprising the 11 N-terminal amino acids of a the rat zeta-chain, wherein said 11 N-terminal amino acids consist of the sequence QSFGLLDPKLC, wherein said nucleic acid sequence encodes a V_{H} chain or wherein said nucleic acid sequence encodes a V_{L} chain. Said intact cells are preferably T-cells or NK-cells or precursors thereof, but may also be artificially transfected cells. In accordance with the present invention, the antibody comprising said CDR is obtainable by priming rats with Jurkat (ATCC TIB-152) cells and boosting them with a conjugate comprising a carrier molecule and the above recited peptide. It is also envisaged that alternative immunization strategies may be successful such as injecting, for example, rats with one or more doses of the conjugate. Whereas in generating the antibody KLH has been used as a carrier, different carriers might be successfully employed. An example of a different carrier is BSA.

Since zeta-chain molecules occur on the surface of T-cells and NK-cells either in association with the TCR and CD3 or with Fcγ-RIIIA or possibly as free floating homo- or heterodimers, said interaction may be with either of these forms of zeta or with all of them. Similarly, the antibody may interact with a single zeta chain or specifically with the dimeric structure. Since eta has the same extracellular domain in humans as zeta, the antibody of the invention also recognizes eta in the same conformation and/or associations as the zeta-chain. Further, the antibody will also specifically interact with the extracellular domain of the zeta/eta-chains of rats and mice.

A particularly advantageous property of the antibody of the invention is the fact that it recognizes zeta/eta both on T-cells and NK-cells as well as precursors thereof. In view of what was known about the structure and associations of the zeta chain, the development of such an antibody has indeed to be considered as surprising. Epitopes on the extremely short, 9 amino acid peptide are necessarily located very close to the cell membrane. The close structural and hence spatial association with multimolecular protein complexes on the cell surface make it seem likely, that these epitopes will also be sterically inaccessible for such a large structure as an antibody. Furthermore, due to the association of the zeta-chain with different multimolecular protein complexes on T-lymphocytes and NK-cells, extracellular zeta chain epitopes unexpectedly accessible for antibody molecules on T-cells are unlikely to be identical with those on NK-cells. In addition, due to the sequence identity in humans, rats and mice, the extracellular zeta-chain domain represents a self-antigen in all three species, thus making it unlikely to obtain specific antibodies against it by immunization of mice and rats.

The above notion that the development of this antibody must be regarded as highly surprising is corroborated by the fact that the most promising approach to obtain such an antibody failed. Namely, a combinatorial antibody library cloned from the RNA of spleen cells of mice immunized with the peptide-KLH conjugate was displayed on filamentous phage and selected in vitro by alternate panning on peptide-BSA conjugate, purified CDB⁺-T-lymphocytes and purified NK-cells. Although enrichment of phage clones displaying Fab-antibody fragments reactive with the peptide-BSA conjugate was attained during the last panning step on CD8⁺-T-lymphocytes, most of them were lost during the subsequent panning step on purified NK-cells, thus indicating the lack of antibodies within the repertoire that recognize a common extracellular zeta-chain epitope on T-lymphocytes and NK-cells. This was confirmed by testing a large number of clones that were reactive with the peptide-BSA conjugate, of which none could be identified with crossreactive binding activity on T-lymphocytes and NK-cells.

Only when the inventors applied an old-fashioned and rather cumbersome approach to the generation of such an antibody, they were eventually successful. This approach comprised the following steps: A peptide comprising the 11 first N-terminal amino acids of the zeta-chain was synthesized and coupled to KLH via the SH-group on cystein 11. Rats preimmunized with Jurkat cells and mice were immunized with this conjugate, respectively, and hybridoma cell lines obtained were screened against another conjugate consisting of the 11-amino acid peptide coupled to BSA. 150 murine and 45 rat hybridoma cell lines were obtained that recognized the peptide-BSA conjugate, of which only one rat IgM antibody could be identified that exhibited binding activity to both T-lymphocytes and NK-cells as determined by flow cytometry.

The antibodies of the present invention or their corresponding immunoglobulin chain(s) can be further modified using conventional techniques known in the art, for example, by using amino acid deietion(s), insertion(s), substitution(s), addition(s), and/or recombination(s) and/or any other modification(s) known in the art either alone or in combination. Methods for introducing such modifications in the DNA sequence underlying the amino acid sequence of an immunoglobulin chain are well known to the person skilled in the art; see, e.g., Sambrook, Molecular Cloning A Laboratory Manual, Cold Spring Harbor Laboratory (1989) N.Y. The antibody may also be a chimeric antibody.

The nucleic acid molecule of the invention may, for example, be an RNA molecule or a DNA molecule. In a preferred embodiment the nucleic acid molecule of the invention is a DNA molecule. Particularly preferred is a synthetic or semisynthetic DNA molecule.

In a further preferred embodiment of the nucleic acid molecule of the invention said CDR has one of the following nucleotide sequences:
(a) SEQ ID No. 1
(b) SEQ ID No. 3
(c) SEQ ID No. 5
(d) SEQ ID No. 7
(e) SEQ ID No. 9
(f) SEQ ID No. 11

In a particularly preferred embodiment of the nucleic acid molecule of the invention said V_{H}-chain has the nucleotide sequence of SEQ ID No. 13 or encodes the amino acid sequence of SEQ ID No. 14.

In another particularly preferred embodiment of the nucleic acid molecule of the invention said V_{L}-chain has the nucleotide sequence of SEQ ID No. 15 or encodes the amino acid sequence of SEQ ID No. 16.

The invention also relates to the nucleic acid molecule of the invention wherein the CDR encodes one of the amino acids sequences of SEQ ID Nos. 2, 4, 6, 8, 10 or 12.

The invention also relates to a vector comprising the nucleic acid molecule of the invention.
The vector of the invention may comprise further genes such as marker genes which allow for the selection of said vector in a suitable host cell and under suitable conditions. Preferably, the polynucleotide of the invention is operatively linked to expression control sequences allowing expression in prokaryotic or eukaryotic cells.

Expression of said polynucleotide comprises transcription of the polynucleotide into a translatable mRNA. Regulatory elements ensuring expression in eukaryotic cells, preferably mammalian cells, are well known to those skilled in the art. They usually comprise regulatory sequences ensuring initiation of transcription and optionally poly-A signals ensuring termination of transcription and stabilization of the transcript. Additional regulatory elements may include transcriptional as well as translational enhancers, and/or naturally-associated or heterologous promoter regions. In this respect, the person skilled in the art will readily appreciate that the polynucleotides encoding at least the variable domain of the light and/or heavy chain may encode the variable domains of both immunoglobulin chains or only one. Likewise, said polynucleotides may be under the control of the same promoter or may be separately controlled for expression. Possible regulatory elements permitting expression in prokaryotic host cells comprise, e.g., the PL, lac, trp or tac promoter in E. coli, and examples for regulatory elements permitting expression in eukaryotic host cells are the AOX1 or GAL1 promoter in yeast or the CMV-, SV40- , RSV-promoter (Rous sarcoma virus), CMV-enhancer, SV40-enhancer or a globin intron in mammalian and other animal cells. Beside elements which are responsible for the initiation of transcription such regulatory elements may also comprise transcription termination signals, such as the SV40-poly-A site or the tk-poly-A site, downstream of the polynucleotide. Furthermore, depending on the expression system used leader sequences capable of directing the polypeptide to a cellular compartment or secreting it into the medium may be added to the coding sequence of the polynucleotide of the invention and are well known in the art. The leader sequence(s) is (are) assembled in appropriate phase with translation, initiation and termination sequences, and preferably, a leader sequence capable of directing secretion of translated protein, or a portion thereof, into the periplasmic space or extracellular medium. Optionally, the heterologous sequence can encode a fusion protein including an C- or N-terminal identification peptide imparting desired characteristics, e.g., stabilization or simplified purification of expressed recombinant product. In this context, suitable expression vectors are known in the art such as Okayama-Berg cDNA expression vector pcDV1 (Pharmacia), pCDM8, pRc/CMV, pcDNA1, pcDNA3 (In-vitrogene), or pSPORT1 (GIBCO BRL).
Preferably, the expression control sequences will be eukaryotic promoter systems in vectors capable of transforming or transfecting eukaryotic host cells, but control sequences for prokaryotic hosts may also be used. Once the vector has been incorporated into the appropriate host, the host is maintained under conditions suitable for high level expression of the nucleotide sequences, and, as desired, the collection and purification of the immunoglobulin light chains, heavy chains, light/heavy chain dimers or intact antibodies, binding fragments or other immunoglobulin forms may follow; see, Beychok, Cells of Immunoglobulin Synthesis, Academic Press, N.Y., (1979).
The vector of the present invention which may e.g. be a plasmid, cosmid, virus or bacteriophage is preferably an expression vector. Expression vectors derived from viruses such as retroviruses, vaccinia virus, adeno-associated virus, herpes viruses, or bovine papilloma virus, may be used for delivery of the polynucleotides or vector of the invention into targeted cell population. Methods which are well known to those skilled in the art can be used to construct recombinant viral vectors; see, for example, the techniques described in Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (1989). Alternatively, the polynucleotides and vectors of the invention can be reconstituted into liposomes for delivery to target cells. The vectors containing the polynucleotides of the invention (e.g., the heavy and/or light variable domain(s) of the immunoglobulin chains encoding sequences and expression control sequences) can be transferred into the host cell by well-known methods, which vary depending on the type of cellular host. For example, calcium chloride transfection is commonly utilized for prokaryotic cells, whereas calcium phosphate treatment or electroporation may be used for other cellular hosts; see Sambrook, supra.

The invention further relates to a host cell transformed or transfected with the vector of invention.
Said host cell may be a prokaryotic or eukaryotic cell. The polynucleotide or vector of the invention which is present in the host cell may either be integrated into the genome of the host cell or it may be maintained extrachromosomally.
The host cell can be any prokaryotic or eukaryotic cell, such as a bacterial, insect, fungal, plant, animal or human cell. Preferred fungal cells are, for example, those of the genus Saccharomyces, in particular those of the species S. cerevisiae. The term "prokaryotic" is meant to include all bacteria which can be transformed or transfected with a DNA or RNA molecules for the expression of an antibody of the invention or the corresponding immunoglobulin chains. Prokaryotic host cells may include gram negative as well as gram positive bacteria such as, for example, E. coli, S. typhimurium, Serratia marcescens and Bacillus subtilis. The term "eukaryotic" is meant to include yeast, higher plant, insect and preferably mammalian cells. Depending upon the host cell employed in a recombinant production procedure, the (poly)peptides/antibodies or immunoglobulin chains encoded by the polynucleotide of the present invention may be glycosylated or may be non-glycosylated. Antibodies of the invention or the corresponding immunoglobulin chains may also include an initial methionine amino acid residue. A polynucleotide of the invention can be used to transform or transfect the host cell using any of the techniques commonly known to those of ordinary skill in the art. Furthermore, methods for preparing fused, operably linked genes and expressing them in, e.g., mammalian cells and bacteria are well-known in the art (Sambrook, Molecular Cloning: A Laboratory Manual, Cold Spring Harbor Laboratory, Cold Spring Harbor, NY, 1989). The genetic constructs and methods described therein can be utilized for expression of the (poly)peptde/antibody of the invention or the corresponding immunoglobulin chains in eukaryotic or prokaryotic host cells. In general, expression vectors containing promoter sequences which facilitate the efficient transcription of the inserted polynucleotide are used in connection with the host cell. The expression vector typically contains an origin of replication, a promoter, and a terminator, as well as specific genes which are capable of providing phenotypic selection of the transformed cells. Furthermore, transgenic animals, preferably mammals, comprising cells of the invention may be used for the large scale production of the (poly)peptide of the invention.
The transformed host cells can be grown in fermentors and cultured according to techniques known in the art to achieve optimal cell growth. Once expressed, the whole (poly)peptides/antibodies, their dimers, individual light and heavy chains, or other immunoglobulin forms of the present invention, can be purified according to standard procedures of the art, including ammonium sulfate precipitation, affinity columns, column chromatography, gel electrophoresis and the like; see, Scopes, "Protein Purification", Springer-Verlag, N.Y. (1982). The antibody or its corresponding immunoglobulin chain(s) of the invention can then be isolated from the growth medium, cellular lysates, or cellular membrane fractions. The isolation and purification of the, e.g., microbially expressed antibodies or immunoglobulin chains of the invention may be by any conventional means such as, for example, preparative chromatographic separations and immunological separations such as those involving the use of monoclonal or polyclonal antibodies directed, e.g., against the constant region of the antibody of the invention. It will be apparent to those skilled in the art that the antibodies of the invention can be further coupled to other moieties for, e.g., drug targeting and imaging applications. Such coupling may be conducted chemically after expression of the (poly)peptide/antibody or antigen to site of attachment or the coupling product may be engineered into the (poly)peptide/antibody of the invention at the DNA level. The DNAs are then expressed in a suitable host cell system, and the expressed proteins are collected and renatured, if necessary.

The invention relates further to a method of producing a (poly)peptide encoded by the nucleic acid molecule of the invention comprising culturing the host cell of the invention under suitable conditions and isolating said (poly)peptide from the culture.
Culturing of said host cells is, in general, described above and may be effected according to established protocols. The same holds true for the isolation of the (poly)peptides.

Additionally, the invention relates to a (poly)peptide that is encoded by the nucleic acid molecule of invention or produced by the method of the invention.

The invention also relates to an antibody or fragment or derivative thereof comprising at least one (poly)peptide of the invention.
Preferably, the antibody of the invention comprises both the complete above-referenced V_{H} and V_{L} chains in conjunction with appropriate constant regions such as µ, γ, α, κ or λ chain.

Specific applications of the antibody or fragment or derivative of the invention include the following:
Mature T-lymphocytes may be functionally affected by structural or functional TCR-blockage as a result of antibodies or antibody derivatives specifically bound to the zeta-chain or by virtue of biologically or pharmaceutically active molecules targeted to the T-cell surface by anti-zeta chain antibodies or antibody fragments/derivatives.
Furthermore, development and selection of thymocytes may be affected by structural or functional TCR- or pre-TCR-blockage as a result of antibodies or antibody fragments/derivatives specifically bound to the zeta chain.
As the zeta chain is consistently expressed during the whole T-cell development from the most immature thymocytes to the mature T-lymphocytes the molecule may be useful for targeting a broad range of T-cell malignancies.
NK-cells may also be functionally affected by structural or functional FcγRIIIA-blockage as a result of antibodies or antibody derivatives specifically bound to the zeta-chain or by virtue of biologically or pharmaceutically active molecules targeted to the NK-cell surface by anti-zeta chain antibodies or antibody fragments or derivatives thereof.

The antibody or fragments or derivatives thereof may, inter alia, be a (semi)synthetic or a classically developed monoclonal antibody. Fragments include Fab' or F(ab)₂ fragments.

The antibodies of the present invention can comprise a further domain, said domain being linked by covalent or non-covalent bonds. The linkage can be based on genetic fusion according to the methods known in the art and described above or can be performed by, e.g., chemical cross-linking as described in, e.g., WO 94/04686. The additional domain present in the fusion protein comprising the antibody of the invention may preferably be linked by a flexible linker, advantageously a polypeptide linker, wherein said polypeptide linker comprises plural, hydrophilic, peptide-bonded amino acids of a length sufficient to span the distance between the C-terminal end of said further domain and the N-terminal end of the antibody of the invention or vice versa. The above described fusion protein may further comprise a cleavable linker or cleavage site for proteinases. These spacer moieties, in turn, can be either insoluble or soluble (Diener, et al., Science, 231:148, 1986) and can be selected to enable drug release from the antigen at the target site. Examples of therapeutic agents which can be coupled to the antibodies of the invention for immunotherapy are drugs, radioisotopes, lectins, and toxins. The drugs with which can be conjugated to the antibodies of the invention include compounds which are classically referred to as drugs such as mitomycin C, daunorubicin, and vinblastine.
In using radioisotopically conjugated antibodies of the invention for, e.g., immunotherapy, certain isotopes may be more preferable than others depending on such factors as leukocyte distribution as well as stability and emission. Depending on the autoimmune response, some emitters may be preferable to others. In general, α and β particle-emitting radioisotopes are preferred in immunotherapy. Preferred are short range, high energy α emitters such as ²¹²Bi. Examples of radioisotopes which can be bound to the antibodies, antigens or epitopes of the invention for therapeutic purposes are ¹²⁵I, ¹³¹I, ⁹⁰Y, ⁶⁷Cu, ²¹²Bi, ²¹²At, ²¹¹Pb, ⁴⁷Sc, ¹⁰⁹Pd and ¹⁸⁸Re. Other therapeutic agents which can be coupled to the antibody, antigen or epitope of the invention, as well as ex vivo and in vivo therapeutic protocols, are known, or can be easily ascertained, by those of ordinary skill in the art. Wherever appropriate the person skilled in the art may use a polynucleotide of the invention encoding any one of the above described antibodies, antigens or epitopes or the corresponding vectors instead of the proteinaceous material itself.

In a preferred embodiment, the antibody of the invention is a monoclonal antibody.

In another preferred embodiment of the invention the antibody the invention is a bispecific antibody.

In a preferred embodiment of the bispecific antibody of the invention, the first specificity is for the extracellular domain of the human zeta-chain on the surface of an intact cell and the second specificity is for an optionally different molecule on the surface of a T-lymphocyte, a natural killer cell or a precursor thereof.
The bispecific antibody of the invention may bind to the above-referenced targets which may be located on the same cell or on different cells. Said different cells may be, for example, two different T-lymphocytes of the same type or a T-lymphocyte and a natural killer cells or precursors of the above, respectively.

In another preferred embodiment of the bispecific antibody of the invention the first specificity is for the extracellular domain of the human zeta-chain on the surface of an intact cell and the second specificity is for a different molecule on the surface of a different cell, preferably of a cell different from a T-cell, an NK-cell or a precursor thereof. Preferably this molecule is a virus encoded antigen, a tumor associated antigen or a surface antigen either on antigen presenting cells (APCs), most preferably dendritic cells, or on non-APCs.
A preferred application of the bispecific antibody of the invention is to redirect T-lymphocytes against target cells by simultaneously targeting the zeta chain and a target cell surface antigen with a bispecific antibody instead of directing T-lymphocytes on target cells by transfecting them with a chimeric zeta-chain receptor (Romeo, Cell 64 (1991) 1037-1046). Since the bispecific antibody binds to native zeta chain, which is associated with the other TCR-subunits, the signaling machinery of the whole TCR-complex may thus be recruited. In contrast, chimeric zeta-chain receptors do not associate with endogenous TCR-subunits and may thus only recruit the isolated signaling effect of the zeta-chain, which seems to be insufficient to activate resting T-cells or may cause unbalanced alteration of the functional state of T-lymphocytes with respect to the induction of activation versus apoptosis.
A preferred application of T-cell retargeting comprises the lysis of target cells by directing on them the cytotoxic activity of cytotoxic T-lymphocytes. Another preferred application of T-cell retargeting comprises the priming of naive T-lymphocytes by crosslinking of their zeta-chain molecules with a surface antigen on antigen presenting cells (APC) or on non-APCs which have been modified to provide sufficient costimulatory signals. On the other hand naive T cells may be anergized or depleted by zeta-chain mediated retargeting on cells that do not provide sufficient costimulatory signals.
Another preferred application of T-cell retargeting comprises the induction of apoptosis in mature activated T-lymphocytes by strong zeta chain mediated TCR reengagement thus imitating mechanisms that mediate peripheral T-cell tolerance and contribute to peripheral immune homeostasis.
Thymic selection of thymocytes during T-cell development may be modified by bispecific antibodies through crosslinkage of the zeta chain on thymocytes with surface molecules on thymic antigen presenting cells directly involved in the positive and negative selection process.

A further preferred application of the bispecific antibody of the invention comprises the retargeting of the cytotoxic activity of NK-cells against target cells by simultaneously targeting the zeta-chain and a target cell surface antigen with a bispecific antibody instead of directing NK-cells on target cells by transfection of a chimeric zeta-chain receptor (Tran, J. Immunol. 155 (1995) 1000-1009).

In a further preferred embodiment the derivative of the antibody of the invention is an scFv chain.

In a preferred embodiment the monoclonal antibody of the invention, said antibody is an IgM.

The invention relates further to a bispecific receptor comprising a (poly)peptide of the invention and natural receptor, natural ligands or a derivative thereof interacting with a surface molecule on the same or on another cell; preferably said receptors or ligands are CD4, CTLA-4, B7-1, B7-2, LFA-3, ICAM-1, -2, -3 or chemokines like MIP-1α, MIP-1β, RANTES or SDF-1.
Applications of the bispecific antibody of the invention are envisaged to also apply for the bispecific receptor of the invention.

The invention relates further to a pharmaceutical composition comprising the nucleic acid molecule of the invention, the vector, the host cell, the (poly)peptide, the antibody or fragment or derivative thereof and/or the bispecific receptor of the invention.

The pharmaceutical composition of the present invention may further comprise a pharmaceutically acceptable carrier. Examples of suitable pharmaceutical carriers are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. Administration of the suitable compositions may be effected by different ways, e.g., by intravenous, intraperitoneal, subcutaneous, intramuscular, topical or intradermal administration. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. A typical dose can be, for example, in the range of 0.001 to 1000 µg (or of nucleic acid for expression or for inhibition of expression in this range); however, doses below or above this exemplary range are envisioned, especially considering the aforementioned factors. Generally, the regimen as a regular administration of the pharmaceutical composition should be in the range of 1 µg to 10 mg units per day. If the regimen is a continuous infusion, it should also be in the range of 1 µg to 10 mg units per kilogram of body weight per minute, respectively. Progress can be monitored by periodic assessment. Dosages will vary but a preferred dosage for intravenous administration of DNA is from approximately 10⁶ to 10¹² copies of the DNA molecule. The compositions of the invention may be administered locally or systemically. Administration will generally be parenterally, e.g., intravenously; DNA may also be administered directly to the target site, e.g., by biolistic delivery to an internal or external target site or by catheter to a site in an artery. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. Furthermore, the pharmaceutical composition of the invention may comprise further agents such as interleukins or interferons depending on the intended use of the pharmaceutical composition.

It is envisaged by the present invention that the various polynucleotides and vectors of the invention are to be administered either alone or in any combination using standard vectors and/or gene delivery systems, and optionally together with a pharmaceutically acceptable carrier or excipient. Subsequent to administration, said polynucleotides or vectors may be stably integrated into the genome of the subject. On the other hand, viral vectors may be used which are specific for certain cells or tissues and persist in said cells. Suitable pharmaceutical carriers and excipients are well known in the art.

Furthermore, it is possible to use a pharmaceutical composition of the invention which comprises polynucleotide or vector of the invention in gene therapy. Suitable gene delivery systems may include liposomes, receptor-mediated delivery systems, naked DNA, and viral vectors such as herpes viruses, retroviruses, adenoviruses, and adeno-associated viruses, among others; see also supra. Delivery of nucleic acids to a specific site in the body for gene therapy may also be accomplished using a biolistic delivery system, such as that described by Williams (Proc. Natl. Acad. Sci. USA 88 (1991), 2726-2729).

The pharmaceutical compositions and uses of the present invention may be desirably employed in humans as well as in animals.

The invention also relates to the use of the antibody of the invention wherein the first specificity is for the extracellular domain of the human zeta-chain and the second specificity is for a different molecule on the surface of a T-lymphocyte, a natural killer cell or a precursor thereof for the preparation of a pharmaceutical composition for the treatment or prevention of autoimmune diseases, immune deficiencies, T-cell malignancies, infectious diseases or for the suppression of immune response especially in order to avoid graft rejection after organ transplantation.
Beside the elimination of target cells (e.g. tumor cells or virus infected cells) the modes described in the present invention of engaging (normal or malignant) cells of the T-cell lineage by extracellular targeting of the zeta chain may be therapeutically used to enhance or suppress immune responses and/or to influence T-cell disorders related to autoimmune diseases, immunodeficiencies or T-cell malignancies. Immune suppression based on the extracellular targeting of the zeta- (and eta-) chain may be preferably used to prevent graft rejection after transplantation.
The modes described in the present invention of modifying signal transduction during T cell development and thymocyte selection by extracellular targeting of the zeta chain may be therapeutically used to enhance favorable immune responses e.g. in case of infectious diseases, tumors and immunodeficiencies or to suppress unfavorable immune responses e.g. in case of autoimmune diseases or graft rejection after transplantation.

Further, the invention relates to the use of the antibody of the invention wherein the first specificity is for the extracellular domain of the human zeta-chain and the second specificity is for a different molecule on the surface of a different cell for the preparation of a pharmaceutical composition for the treatment or prevention of malignancies, viral infections and other infectious diseases.

The invention in addition relates to the use of the (poly)peptide or the antibody or fragment or derivative thereof or the bispecific receptor of the invention for the preparation of a pharmaceutical composition for the enhancement or suppression of NK-cell dependent immunity or for the treatment of NK-cell derived malignancies.
Beside the elimination of target cells (e.g. tumor cells or virus infected cells) the described modes of engaging NK-cells by extracellular targeting of the zeta chain may be therapeutically used to enhance or suppress NK-cell dependent immunity or to influence NK-cell derived malignancies. As the zeta-chain is expected to be also expressed on NK-cell derived malignancies, the molecule may furthermore be useful for targeting malignant NK-cells.

In addition the invention relates to an in vitro method for the determination of zeta-chain or eta-chain expression on NK-cells, T-lymphocytes or precursors thereof comprising
(a) contacting the (poly)peptide or the antibody or fragment or derivative thereof of the invention with said NK-cells, T-lymphocytes or precursors thereof; and
(b) assessing the amount of bound (poly)peptide, antibody or derivative.

Contacting can be carried out by incubating preferably on ice said (poly)peptide or said antibody or fragment or derivative thereof with said NK-cells, T-lymphocytes or precursors thereof in a biological buffer resembling physiological conditions (e.g. phosphate-buffered saline, PBS) for 20 to 40 minutes. After two washing steps with PBS bound (poly)peptide, antibody or fragment or derivative thereof can be detected for example with an appropriate fluorescent-labeled secondary antibody and quantitated by flow cytometric analysis as described in Example 4.

The invention relates further to a kit comprising the nucleic acid molecule, the vector, the host cell, the (poly)peptide, the antibody or fragment or derivative thereof and/or the bispecific receptor of the invention.

Finally, the invention relates to a non-human transgenic animal comprising in its germline at least one copy of the nucleic acid molecule or the vector of the invention.
Non-human transgenic animals may be produced according to conventional protocols as described, for example in Palmiter R.D., Brinster R.L.: Germline transformation of mice. Ann. Rev. Genet. 20 (1986), 465-499 and Capecci M.: Altering the genome by homologous recombination. Science 244 (1991) 1288-1292. Preferred examples of the transgenic animals of the invention are cows, sheep, rabbits, mice or rats.

Table 1 shows primer sets for the PCR-amplification of murine lg-heavy and light chain-DNA-fragments

The figures show:

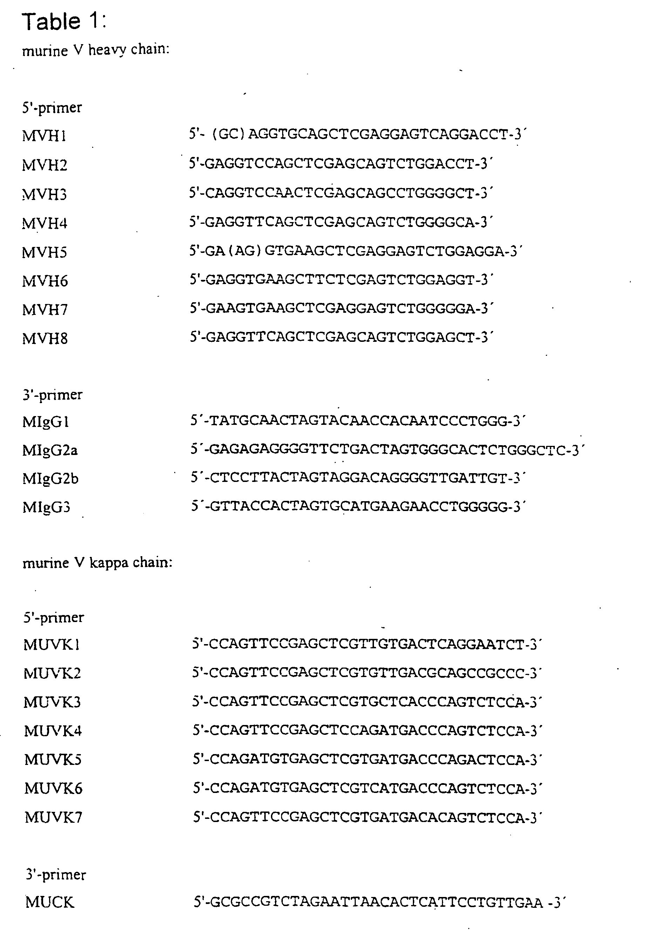

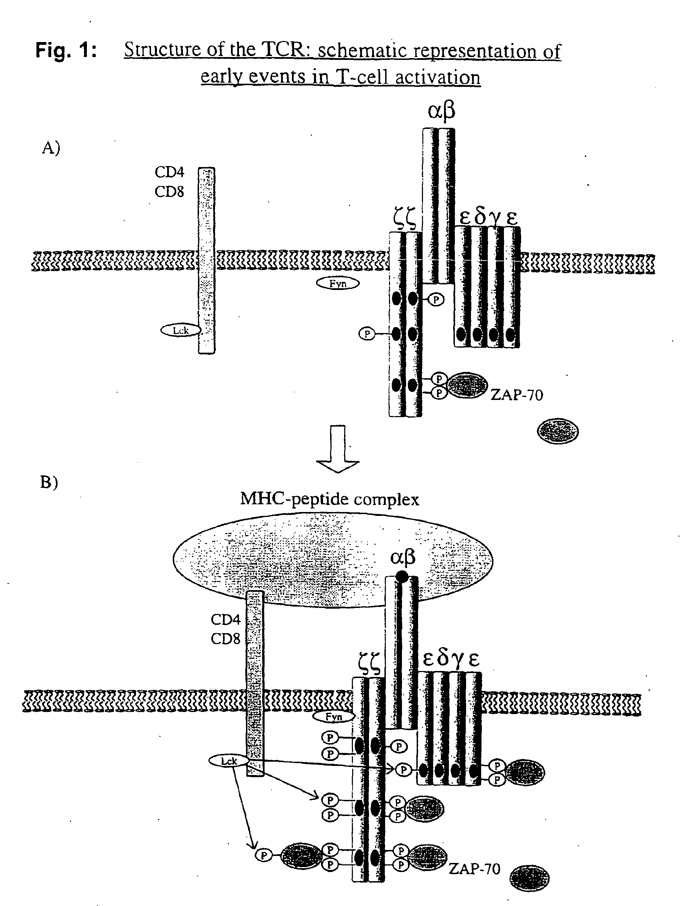

**Figure 1:** Structure of the TCR and early events in T-cell activation. The TCR consists of clonotypic chains (α + β) and invariant chains (ζ, CD3γ, CD3δ, and CD3ε) with the probable subunit composition TCR α + β, CD3 εδγε, ζζ. The location of the ITAMs within the cytoplasmatic domains of the CD3 and ζ chains are shown as small black ovals. **A)** In resting T-cells, ITAMs are either non-phosphorylated or only partially phosphorylated. The protein tyrosine kinase Lck, is associated with the cytoplasmic domain of CD4 or CD8. **B)** Upon interaction with an MHC-peptide-complex, the TCR and either CD4 or CD8 are co-aggregated and the ITAMs within the CD3 and ζ chains are phosphorylated by the Src kinases Lck and/or Fyn. ZAP-70 or the related kinase, Syk, binds specifically to ITAMs in which both tyrosine residues have been phosphorylated. Following its recruitment to the TCR-complex, ZAP-70 is activated by Lck, subsequently after other molecules are thus recruited to the TCR-complex and activation proceeds to further downstream molecules (not shown). Circled P represents phosphorylated tyrosine residues.

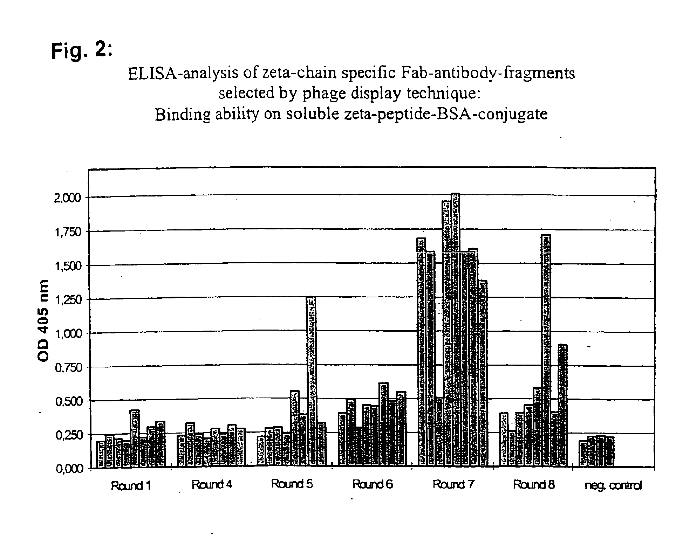

**Figure 2 :** ELISA-analysis of murine Fab-antibody-fragments selected by phage display for binding to the extracellular part of the human zeta-chain. Periplasma preparations of soluble Fab-fragments expressed in E. *coli* were incubated with immobilized zeta-peptide-BSA-conjugate. Specifically bound Fab-fragments were detected with a horse radish peroxidase conjugated F(ab')₂ fragment of a goat anti-mouse IgG + IgM antibody. The ELISA was developed by adding an ABTS-substrate solution. Eight clones per round of in vitro selection are presented on the x-axis; OD-values were measured by an ELISA-reader at 405 nm and are presented on the y-axis. For negative controls, the wells were incubated with PBS instead of periplasma preparations.

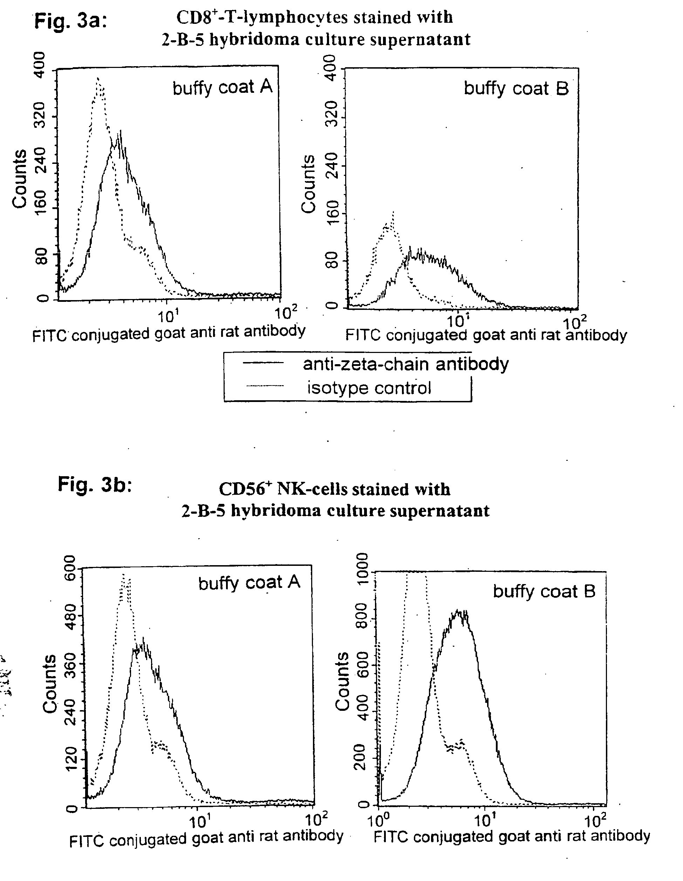

**Figure 3:** Flowcytometry-analysis of the zeta-chain specific binding activity of the 2-B-5 antibody on the surface of CD8⁺-T-lymphocytes and NK-cells. 100.000 mononucleated cells from peripheral blood of two different healthy donors were incubated with undiluted cell culture supernatant of the 2-B-5 hybridoma. Bound zeta-chain specific rat antibody was detected by a fluorescein (FITC) conjugated goat-anti-rat Ig (IgG + IgM) antibody diluted 1:100 in PBS. Triple color fluorescence analysis was carried out by applying a positive gate for CD8⁺ (Tricolor) and a negative gate for CD16⁺ (PE) cells thus allowing the detection of FITC-mediated fluorescence (filled lines) exclusively attributed to CD8⁺-T-lymphocytes (phenotype: CD8⁺, CD16⁻) without any contaminating signals from CD8⁺-NK-cells. Similarly, triple color fluorescence analysis was carried out by applying a positive gate for CD56⁺- (PE) and a negative gate for CD3⁺-cells (tricolor) thus allowing the detection of FITC-mediated fluorescence (filled lines) exclusively attributed to NK-cells (phenotype: CD56⁺, CD3⁻) without any contaminating signals from CD56⁺-T-lymphocytes. As isotype control (broken lines) culture supernatant of an antibody with the same isotype (rat IgM) but irrelevant specificity was used. For the fixation of labeled cells 1% paraformaldehyde in PBS was used. Cells were analyzed by flowcytometry on a FACS-scan (Becton Dickinson).

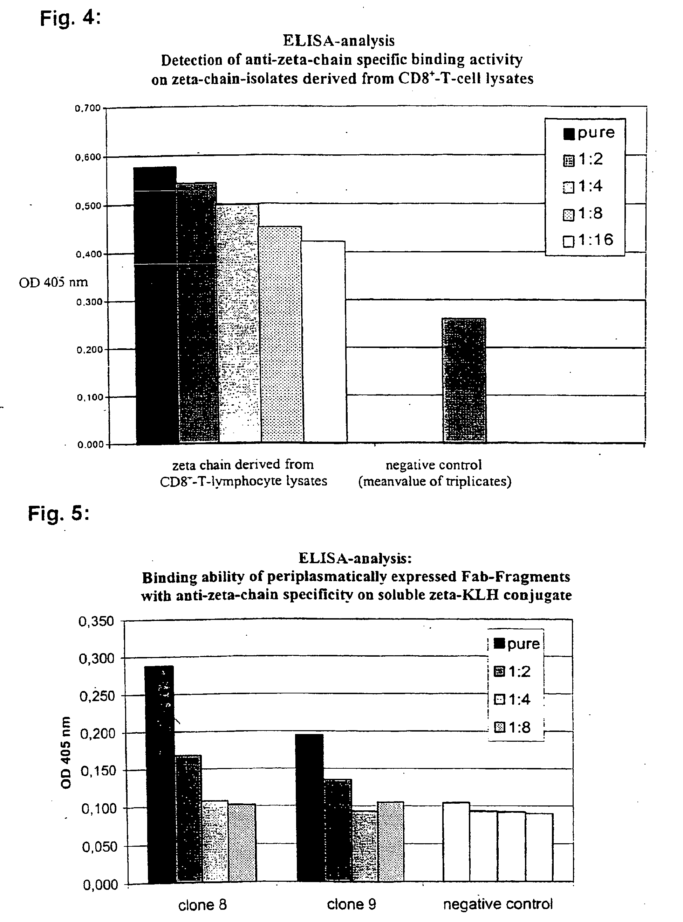

**Figure 4:** Results of a Sandwich-ELISA confirming the reactivity of antibody 2-B-5 with native zeta-chain present in the lysate of the CD8⁺-T-cell lymphocytes. A zeta-chain specific antibody, which recognizes the amino acids 144-163 at the carboxy-terminus of the human zeta chain was coated for 12 hours to the wells of a 96 U-bottom plate, followed by blockade for one hour at room temperature with PBS/3% BSA. Subsequently the lysate of CD8⁺-cells was added undiluted and in several dilutions and incubated for one hour at room temperature. As negative control, the wells were incubated with PBS instead of the cell lysate. In the following step the purified antibody 2-B-5 was added at a concentration of 1 µg/ml and incubated for one hour. Bound 2-B-5 antibody was detected with a biotinylated mouse-anti-rat IgM antibody followed by an Avidin-peroxidase-conjugate. The ELISA was finally developed by addition of ABTS-substrate solution. The colored precipitate was measured at 405 nm using an ELISA-reader.
**Figure 5:** ELISA-based analysis for specific binding to zeta-peptide-KLH-conjugate of recombinant Fab-fragment of the rat monoclonal antibody 2-B-5 expressed in the periplasma of E. *coli*. Coating of zeta-peptide-KLH-conjugate was carried out at 4°C for 12 hours followed by a single washing step with PBS/0,05% Tween. The wells were blocked for 1 hour with PBS/3% bovine serum albumin (BSA) and washed again once. Then Fab-containing periplasma preparations were added undiluted and in several dilutions and incubated for 2 hours. As negative controls, wells were incubated with PBS instead of periplasma preparations. For detection of Fab-fragments bound to the zeta-peptide-KLH-conjugate a murine anti-His-tag antibody was used followed by a peroxidase conjugated polyclonal goat anti-mouse IgG antibody. The ELISA was finally developed by addition of ABTS-substrate solution. The turnover of colored substrate was measured by an ELISA-Reader at OD 405 nm.

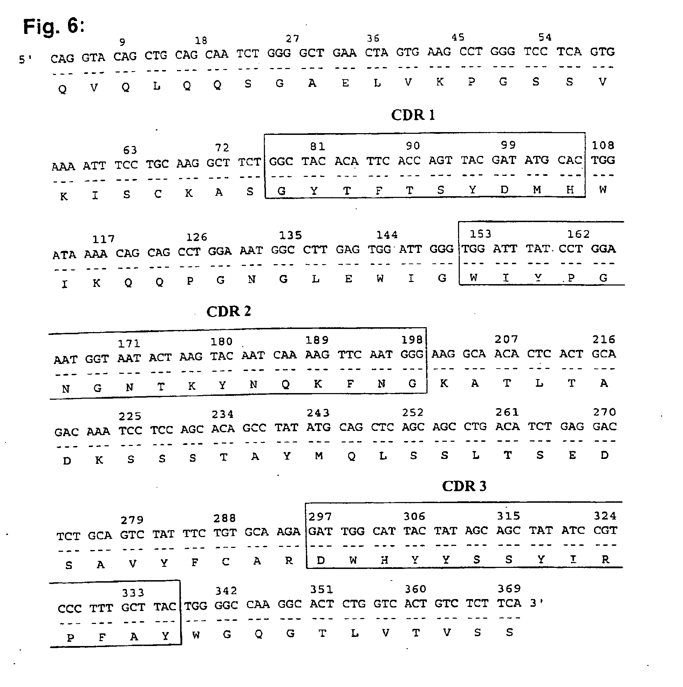

**Figure 6:** DNA- and protein-sequence of the VH-region of the anti-zeta-chain antibody 2-B-5. Numbers indicate the nucleotide (nt) positions, amino acids (aa) are presented in single letter code. Boxes indicate the three CDR's.

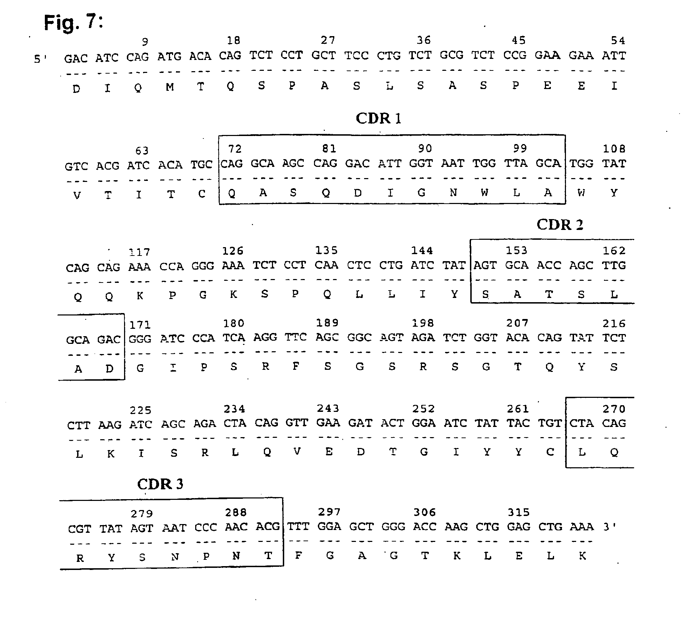

**Figure 7:** DNA- and protein-sequence of the VK-region of the anti-zeta-chain antibody 2-B-5. Numbers indicate the nucleotide (nt) positions, amino acids (aa) are presented in single letter code. Boxes indicate the three CDR's.

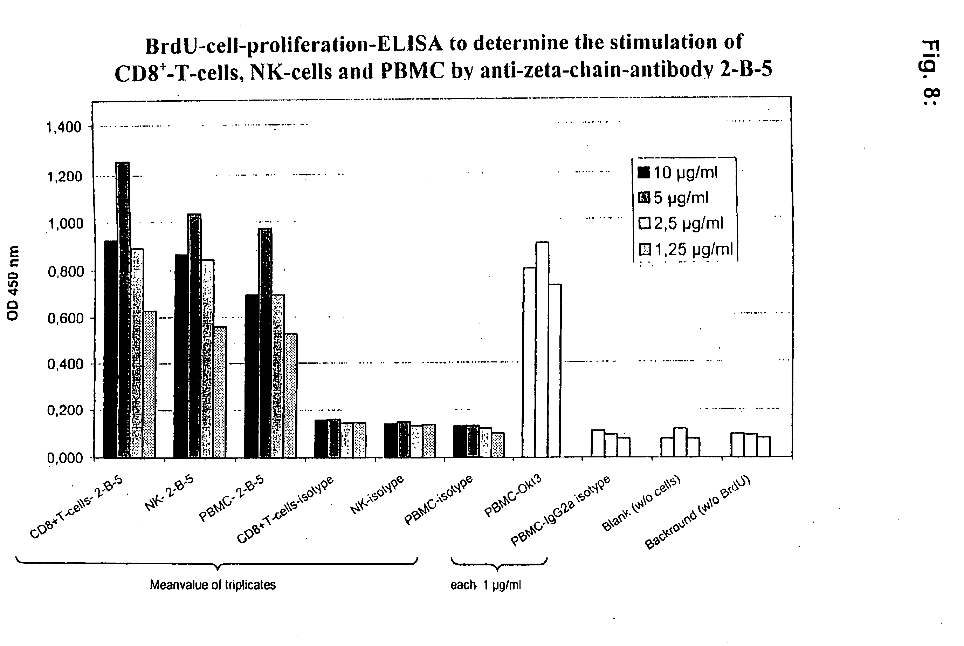

**Figure 8:** Results of an ELISA-based BrdU-incorporation-assay detecting cell proliferation carried out in order to determine the stimulation of CD8⁺-T-cells, NK-cells and PBMC induced by the anti-zeta-chain-antibody 2-B-5. A 96-well flat-bottom microtiterplate was coated with purified 2-B-5 antibody in several dilutions overnight at 4°C. 100.000 CD8⁺-T-lymphocytes, NK-cells and unseparated PBMC were added in triplicates to the wells of a microtiterplate, respectively. To control the specificity of the stimulation mediated by 2-B-5 an antibody of the same isotype (rat IgM) with irrelevant specificity was used at the same concentrations. The antibody OKT3 (Isotype IgG2a), which recognizes the human CD3-complex was applied as specific positive control for the stimulation of T-cells and unseparated PBMC, respectively. A murine IgG2a-antibody of irrelevant specificity was used as isotype control for OKT3. A blank control (wells without cells) and a background control (wells without BrdU) were also included. After incubation period of three days the BrdU-Iabeling solution was added for 24 hours. Subsequently cells were lysed and fixed followed by the addition of an anti-BrdU-antibody, which binds to the BrdU incorporated in newly synthesized, cellular DNA. This peroxidase conjugated antibody was detected by the subsequent substrate reaction. The reaction product was quantified by an ELISA reader at a wavelength of 450 nm.

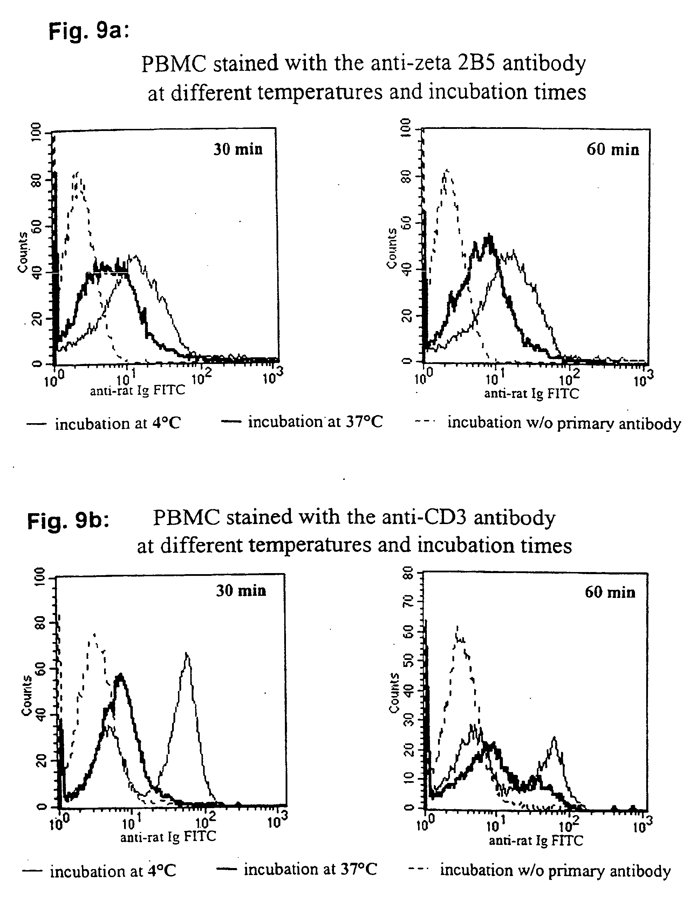

**Figure 9:** Flowcytometric analysis of TCR/CD3 complex internalization induced by the binding of the anti-zeta-chain antibody. 200.000 mononucleated cells were incubated with the anti-zeta-chain antibody 2-B-5 at a concentration of 1 µg/ml, at 4°C or 37°C for either 30 or 60 minutes; a parallel experiment was carried out with a rat-anti-human CD3 antibody. The capping process was terminated by washing twice with cold PBS. To label the cell surface bound antibody, the cells were incubated with a fluorescein (FITC) conjugated goat-anti-rat Ig (IgG + IgM) antibody diluted 1:100 in PBS. As negative control, only the secondary antibody was used.

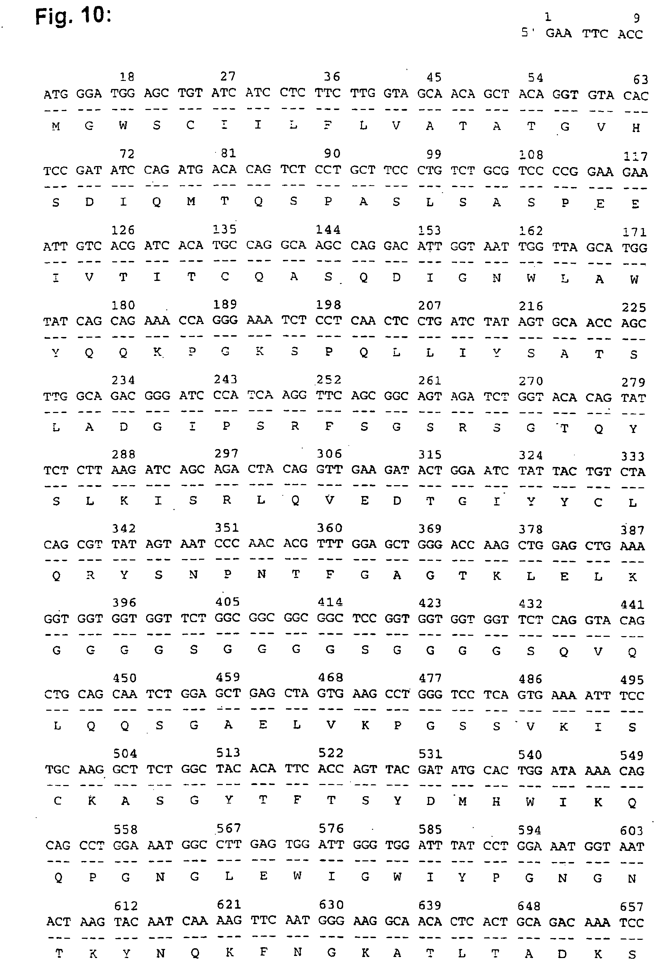

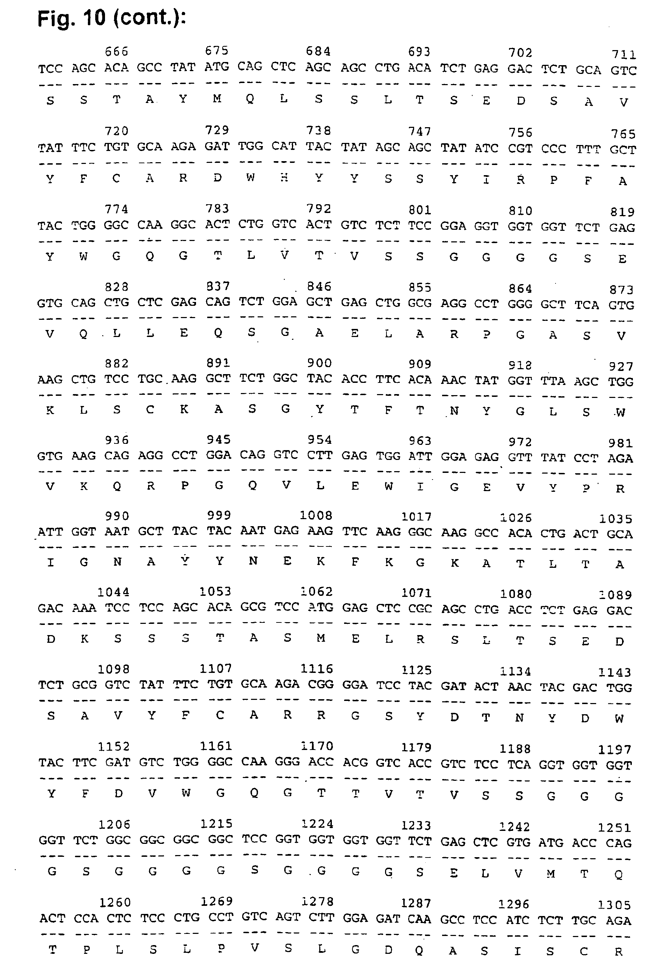

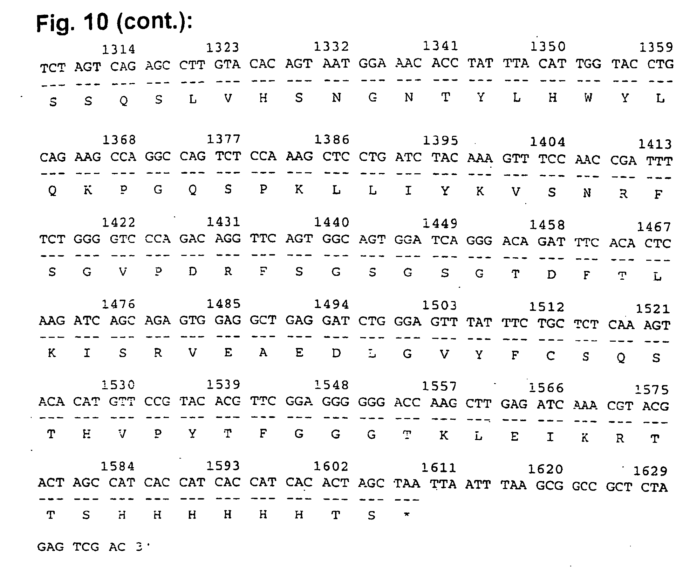

**Figure 10:** DNA- and protein-sequence of the anti-zeta-chain/anti-EpCAM bispecific single-chain antibody. Numbers indicate the nucleotide (nt) positions, the resulting amino acid sequence is depicted below the nucleotide sequence. The DNA sequence encoding the antibody starts at position 67 and ends at position 1605. Nucleotides 10 to 66 encode a leader peptide, that mediates secretion of the bispecific antibody in mammalian cells. The first six nt (position 1 to 6) and the last six nt (position 1632 to 1637) contain the restriction enzyme recognition sites for EcoRI and Sall, respectively.

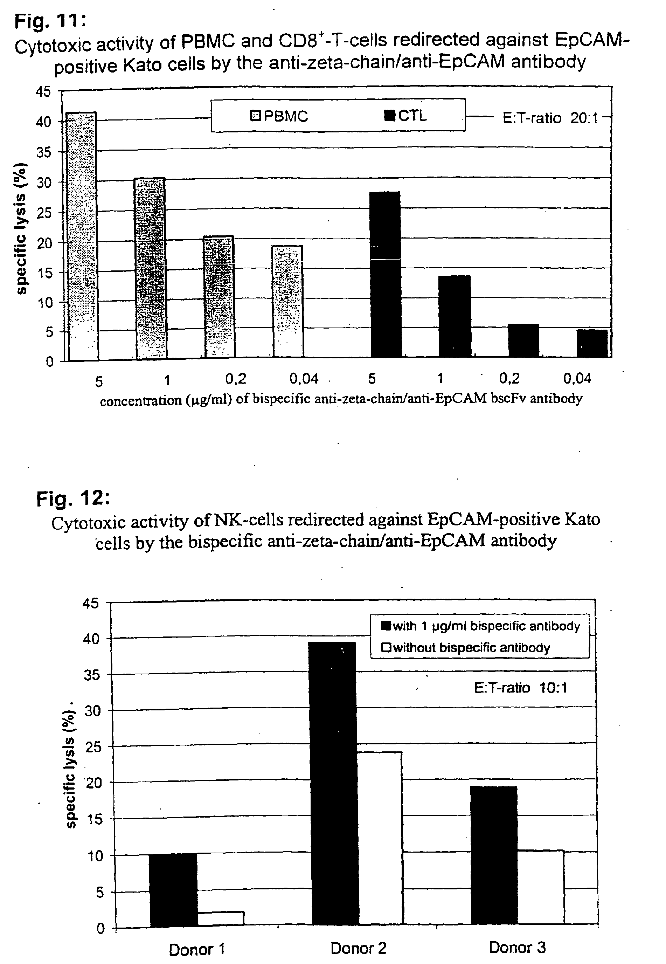

**Figure 11:** Cytotoxic activity of PBMC and CD8+-T-lymphocytes redirected against EpCAM-positive Kato cells by the bispecific anti-zeta-chain/anti-EpCAM antibody. 200.000 unstimulated PBMCs or CD8+ T-lymphocytes in a volume of 100 µl were added to 10.000 Chromium-51 labeled Kato III cells in a volume of 100 µl. The bispecific antibody was added in concentrations from 40 ng/ml to 5 µg/ml in a volume of 50 µl. The microtiterplates were incubated for 16 h at 37° C, 5 % CO2. After the incubation period 50 µl supernatant were removed from each well and assayed for released 51 Cr in a gamma counter.
**Figure 12:** Cytotoxic activity of NK-cells redirected against EpCAM-positive target cells by the bispecific anti-zeta-chain/anti-EpCAM antibody. 100.000 NK-cells in a volume of 100 µl were added to 10.000 Chromium-51 labeled Kato III cells in a volume of 100 µl. The bispecific antibody was added in a concentration of 1 µg/ml in a volume of 50 µl. The microtiterplates were incubated for 4 h at 37° C, 5 % CO2. After the incubation period 50 µl supernatant were removed from each well and assayed for released 51 Cr in a gamma counter.

These and other embodiments are disclosed and encompassed by the description and Examples of the present invention. Further literature concerning any one of the antibodies, methods, uses and compounds may be retrieved from public libraries and databases, using for example electronic devices. For example the public database "Medline" may be utilized which is available on the Internet, for example under http://www.ncbi.nlm.nih.gov/PubMed/medline.html. Further databases and addresses, such as http://www.ncbi.nlm.nih..gov/, http://www.infobiogen.fr/, http://www.fmi.ch/biology/research_tools.html, http://www.tigr.org/, are known to the person skilled in the art and can also be obtained using, e.g., http://www.lycos.com. An overview of patent information in biotechnology and a survey of relevant sources of patent information useful for retrospective searching and for current awareness is given in Berks, TIBTECH 12 (1994), 352-364.

The above disclosure generally describes the present invention. A more complete understanding can be obtained by reference to the following specific examples which are provided herein for purposes of illustration only and are not intended to limit the scope of the invention.

The examples illustrate the invention.

### Example 1: Immunization of mice with zeta-peptide-KLH-conjugate and determination of serum titer using zeta-peptide-BSA-conjugate

Ten weeks old F1 mice from balb/c x C57black crossings were immunized with the zeta-peptide-KLH-conjugate (Jerini Bio Tools, Berlin). The peptide, with the amino acid sequence (QSFGLLDPKLC) of the zeta-chain N-terminus was coupled to the maleinimide activated KLH in directed manner via the mercapto-group of the C-terminal Cystein. The conjugate was dissolved in 0.9% NaCl at a concentration of 100 µg/ml. The solution was subsequently emulsified 1:2 with complete Freund's adjuvants and 50 µl were injected per mouse intraperitonially. Mice received booster immunizations after 4, 8, and 12 weeks in the same way, except that complete Freund's adjuvants was replaced by incomplete Freund's adjuvants. Ten days after the first booster immunization, blood samples were taken and antibody serum titer against zeta-peptide-BSA-conjugate was tested by ELISA. Serum titer was more than 1000 fold higher in immunized than in not immunized animals. Three days after the second boost, spleen cells were fused with P3X63Ag8.653 cells (ATCC CRL-1580) to generate hybridoma cell lines following standard protocols as described in Current Protocols in Immunology (Coligan, Kruisbeek, Margulies, Shevach and Strober, Wiley-Interscience, 1992). After PEG-fusion, cells were seeded at 100.000 cells per well in microtiterplates and grown in 200 µl RPMI 1640 medium supplemented with 10% fetal bovine serum, 300 units/ml recombinant human interleukin 6 and HAT-additive for selection. Culture supernatants from densely grown wells were tested by ELISA-analysis at 1:20 dilution. The ability of the hybridoma supernatants to bind the zeta-peptide-BSA-conjugate was tested by the following ELISA:
100 µl zeta-peptide conjugated to bovine serum albumin in the same way as described for the zeta-peptide-KLH-conjugate (Jerini Bio Tools, Berlin) was coated to wells of a 96 U-bottom plate (Nunc, maxisorb) at a concentration of 5 µg/ml. Coating was performed overnight at 4°C, after washing three times with washing buffer (0.1 M NaCl, 0.05M Na₂HPO₄ pH 7.3, 0.05% Tween 20, 0.05% NaN₃) the following blockade was performed with 200 µl of 2% skimmed milk powder added to the washing buffer for one hour at room temperature. In the next step the hybridoma supernatant was incubated pure and at several dilutions for two hours at room temperature. As detection system diluted horseradish peroxidase conjugated polyclonal antibody against mouse immunoglobulin was used. After 5 times of washing the ELISA was finally developed by addition of TMB-substrate solution (Tetramethylbenzidine, Boehringer Mannheim). The colored precipitate was measured after 15 min. at 405 nm using an ELISA-reader.

Supernatants from 150 clones exhibiting strong ELISA-signals were selected for flowcytometric analysis.

To check the binding activity of the hybridoma supernatants on T-lymphocytes and NK-cells a flowcytometric analysis was performed. 1 x 10⁶ PBMC were incubated for 30 min. on ice in 50 µl undiluted supernatant from 150 different clones, respectively and bound antibodies were detected subsequently by a fluorescein (FITC) conjugated F(ab')₂ fragment of a rabbit anti-mouse Ig antibody (Dako Hamburg, Code No. F0313) diluted 1:100 in PBS. To avoid unspecific binding in the following labeling steps the free valences of the FITC-conjugated antibody were blocked by addition of 50 µl 1:10-diluted mouse serum (Sigma immunochemicals, Deisenhofen, M-5905) for 30 minutes. To distinguish the two PBMC-subsets the previously labeled cells were divided. One half was stained with a 1:100 diluted tricolor conjugated anti-CD8 antibody (Caltac Laboratories; Burlingame; USA, Code No. MHCD0306); the other half was stained with a 1:25 diluted phycoerythrin (PE) conjugated anti-CD56 antibody (Becton Dickinson, Heidelberg, Cat. No. 347747). As negative control murine monoclonal antibody of irrelevant specificity was used instead of the hybridoma supernatants. Unlabeled anti-CD16 and anti-CD6 antibodies for specific staining of NK-cell or T-lymphocytes respectively were used to control the primary labeling step.

Cells were analyzed by flowcytometry on a FACS-scan (Becton Dickinson, Heidelberg). FACS-staining and measuring of the fluorescence intensity were performed as described in Current Protocols in Immunology (Coligan, Kruisbeek, Margulies, Shevach and Strober, Wiley-Interscience, 1992)

Two-color fluorescence analysis was carried out by applying a positive gate for CD8⁺⁻and CD56⁺-cells, respectively thus allowing the detection of FITC-mediated fluorescence separately on CD8⁺-T-lymphocytes and NK-cells. Despite a clear staining of CD8⁺-T-lymphocytes and NK-cells by the respective positive control antibodies, none of the 150 hybridoma supernatants showed binding activity on CD8⁺-T-lymphocytes and/or NK-cells.

### Example 2: In vitro selection for anti-zeta-antibodies of a murine combinatorial antibody library by the phage display method

An F1 mouse from balb/c x C57black crossings was immunized of the age of ten weeks as described in Example 1. Ten days after the first booster immunization blood samples were taken and antibody serum titer against zeta-peptide-BSA-conjugate was tested by ELISA (see Example 1). Serum titer was more than 1000 fold higher in the immunized compared to not immunized animals. Three days after the third injection, murine spleen cells were harvested. For isolating total RNA a protocol according to Chomczynski (Analytical biochemistry 162 (1987) 156-159) was used.
A DNA-library encoding murine immunoglobulin (Ig) kappa light chains and lg heavy chain Fd-fragments (=VH+CH1) was constructed by RT-PCR on murine spleen RNA, respectively. cDNA was synthesized according to standard protocols (Sambrook, Cold Spring Harbor Laboratory Press 1989, second edition).
The primer sets (depicted in Table 1) were chosen, giving rise to a 5'- *XhoI* and a 3'-Spel recognition site for the heavy chain- and to a 5'-*Sac*I and a 3'- *Xba*I recognition site for the light chain fragments. For the PCR-amplification of the HC DNA-fragments eight different 5'-VH-family specific primers were each combined with four 3' primer hybridizing to the 3'-region of the HC-CH1-domain of different IgG-subclasses; for the PCR-amplification of the kappa light chain fragments seven different 5'-VK-family specific primers were each combined with one 3'-primer, hybridizing to the 3'-end of the kappa constant region (CK).
The following PCR program was used for amplification: Initial denaturation at 94°C for 2 min.; 40 cycles of amplification: Denaturation at 94°C for 20 sec.; primer annealing at 52°C for 50 sec. and primer extension at 72 °C for 60 sec., followed by a 10 min. final extension at 72°C.

450 ng of the kappa light chain fragments (*Sac*I-*Xba*I digested) were ligated with 1400 ng of the phagmid pComb3H (*Sac*I*-Xba*I digested; large fragment) (Barbas et al, Proc Natl Acad Sci USA 88, 7978-82 (1991)). The resulting light chain library was then transformed into 300 µl of electrocompetent *Escherichia coli* XL1 Blue by electroporation (2.5 kV, 0.2 cm gap cuvette, 25 FD, 200 Ohm, Biorad gene-pulser) resulting in a library size of 6 x 10⁸ independent clones. After one hour of phenotype expression, positive transformants were selected for the vector encoded carbenicilline resistance in 100 ml of liquid SB-culture over night. Cells were then harvested by centrifugation and plasmid preparation was carried out using a commercially available plasmid preparation kit (Qiagen).

2800 ng of this plasmid-DNA containing the kappa light chain library (*Xho*I-*Spe*I digested; large fragment) were ligated with 900ng of the HC-Fd-DNA-fragments (*Xho*I-*Spe*I digested) and again transformed into two 300 µl aliquots of electrocompetent *E. coli* XL1 Blue by electroporation (2.5 kV, 0.2 cm gap cuvette, 25 FD, 200 Ohm) resulting in a combinatorial library of antibody Fab-fragments consisting of 4 x 10⁸ independent clones.
After one hour of phenotype expression, positive transformation was selected by carbenicilline resistance. After this adaptation these clones were infected with an infectious dose of 1 x 10¹² particles of the helper phage VCSM13 resulting in the production and secretion of filamentous M13 phage, with each Phage particle containing a single-stranded copy of the phagemid vector encoding a single murine antibody Fab-fragment and displaying the corresponding Fab-protein on the phage surface. Fab-fragments were anchored on the phage surface by an translational fusion of the HC-Fd-fragment to the phage coat protein III, with the Ig-light chain spontaneously associating with the HC-fragment.

This phage library carrying the cloned Fab-repertoire was harvested from the culture supernatant by PEG8000/NaCl precipitation and centrifugation, redissolved in RPMI 1640 - medium supplemented with 10% FCS and incubated either with immobilized zeta-peptide-BSA-conjugate or with isolated CD8⁺-lymphocytes or NK-cells in daily alternating order. The two subpopulations of human PBMC were isolated in advance by a immunomagnetic separation method. Mononucleated cells obtained from peripheral blood by Ficoll-density gradient centrifugation were first incubated with a specific primary antibody of murine origin directed against human CD8 or CD56 and subsequently subjected to rosettation with paramagnetic beads (Dynal, Oslo, Norway) conjugated with a sheep-anti-mouse IgG antibody. CD8⁺-T-cells and CD56⁺-NK-cells were isolated with a magnet attached to the wall of the tube containing the cell suspension. After incubation of the phage library with purified CD8⁺-T-lymphocytes or NK-cells for two hours at 4°C under continuous agitation respectively, cell bound phage particles were rescued from unbound phage particles via the paramagnetic beads still attached to the cells.
Accordingly, exposure to the magnetic field was also carried out in order to get rid off washing solution that was applied several times during each round of panning to resuspend the cells and thus reduce the phage background. Specifically bound phage particles were finally eluted from the cells by HCl-Glycine pH 2.2 and after neutralization with 2 M Tris pH 12, the eluate was used for infection of a new uninfected *E. coli* XL1 Blue culture. Cells successfully transduced with a pComb3H phagmid copy, encoding a murine Fab-fragment, were again selected for carbenicilline resistance and subsequently infected with VCMS13 helper phage to start another round of antibody display and in vitro selection.

The complete in vitro selection procedure consisted of two initial rounds of panning on immobilized zeta-peptide-BSA-conjugate followed by one round of panning on CD8⁺-T-lymphocytes and subsequently on CD56⁺-NK-cells respectively. Then another two rounds of panning were carried out on immobilized zeta-peptide-BSA-conjugate again followed by one round of panning on CD8⁺-T-cells and finally on CD56⁺-NK-cells, respectively. Panning on immobilized antigen was performed as described (Barbas et al, Proc Natl Acad Sci USA 88, 7978-82 (1991) in order to keep the selection pressure on zeta-chain specific Fab-fragments that may otherwise be lost by unspecific elution of phage particles, from cell surfaces containing a big number of different antigens in addition to the zeta chain. After each round of panning, plasmid-DNA was prepared from the resulting E. coli culture.

For the production of soluble Fab-proteins the *gene III* DNA fragment was excised from these preparations of plasmid-DNA (*Spe*I/*Nhe*I), thus destroying the translational fusion of the Fab-segment with the *gene III* protein. After religation, this pool of plasmid DNA was transformed into 100 µl heat shock competent E. *coli* XL1 Blue and plated on Carbenicilline LB-Agar. Single colonies were grown in 10 ml LB-Carb-cultures/20 mM MgCl₂ and Fab-expression was induced after six hours by adding lsopropyl-β-D-thiogalactosid (IPTG) to a final concentration of 1 mM.

These cells were harvested after 20 hours by centrifugation and through four rounds of freezing at -70°C and thawing at 37°C the outer membrane of the bacteria was destroyed by temperature shock so that the soluble periplasmatic proteins including the Fab antibody-fragments were released into the liquid. After elimination of intact cells and cell-debris by centrifugation, the supernatant was tested by ELISA for Fab-antibody-fragments binding to the zeta-peptide-BSA-conjugate.
Detection of Fab-fragments bound to immobilized zeta-peptide-BSA-conjugate was carried out using a horse radish peroxidase conjugated F(ab')₂ fragment of a goat anti-mouse IgG + IgM antibody (0,16µg/ml) (Pierce, Rockford, USA, Prod. No. 311448). The signal was developed by adding a substrate solution, containing 2,2'Azino-bis(3-Ethylbenz-Thiazoline-6-Sulfonic Acid) and Na-perborate and detected at a wavelength of 405 nm.

In contrast to clones taken from the library prior to the in vitro selection, those clones tested after different rounds of panning proved to be positive in the zeta-peptide-ELISA with an overall increasing frequency up to the seventh round of panning, which was carried out with purified CD8⁺-T-cells (Figure 2). However, from the seventh to the eighth round of panning, the latter of which was performed with purified NK-cells, the number of positive clones dropped significantly. This indicates that clones that could be enriched by virtue of their binding activity on T-cells did mostly not correct with NK-cells with the only exception of clone 90 still present after the final panning step on NK-cells.

To check the binding activity of zeta-peptide-reactive Fab-fragments on CD8⁺-T-cells and NK-cells a flowcytometric analysis was performed. 1 x 10⁶ PBMC were incubated in 50 µl undiluted periplasma preparation for 30 min. on ice followed by incubation with a fluorescein (FITC) conjugated F(ab')₂-fragment of a goat anti-mouse IgG + IgM antibody (Jackson Immunoresearch laboratories, West Grove, USA Code No. 115-096-068) diluted 1:100 in PBS. To avoid unspecific binding during the following labeling steps the free valences on the FITC-antibody were blocked by addition of 50 µl 1:10 diluted mouse serum (Sigma immunochemicals, Deisenhofen, M-5905) for 30 minutes. To distinguish the two PBMC-subsets the previously labeled cells were divided. One half was stained with a 1:100 diluted tricolor conjugated anti-CD8 antibody (Caltac Laboratories; USA, Code No. MHCD0306); the other half was stained with a 1:25 diluted phycoerythrin conjugated anti-CD56 antibody (Becton Dickinson, Heidelberg, Cat. No. 347747). As negative control Fab-antibody periplasma preparations of an irrelevant specificity were included. Unlabeled anti-CD16 and anti-CD6 antibodies were used as positive controls for NK-cell and CD8⁺⁻T-lymphocytes, respectively.

Two-color fluorescence analysis was carried out on a FACS scan (Becton Dickinson) by applying a positive gate for CD8⁺ - and CD56⁺-cells, respectively thus allowing the detection of FITC-mediated fluorescence separately on CD8⁺-T-lymphocytes and NK-cells. Labeling of cells and fluorescence measurements were performed as described in Current Protocols in Immunology (Coligan, Kruisbeek, Margulies, Shevach and Strober, Wiley-Interscience, 1992).

Using the above mentioned protocol 60 different clones, which proved to react with the zeta-peptide-BSA-conjugate in the ELISA-analysis, were analyzed on PBMC. Despite clear positive FACS-signals exhibited by the positive controls, none of the periplasma preparations of anti-zeta chain Fab-antibody-fragments could be demonstrated to bind on the surface of CD8⁺-T-lymphocytes of NK-cells. This result may be explained by low affinity interactions of Fab-fragments selected by the panning procedure with the surface of T-cells, which may be sufficient for enrichment during the in vitro selection but below the sensitivity of the flowcytometric analysis. However disappearance of zeta-peptide-reactive clones during the final selection step carried out on NK-cells strongly indicates that these potentially T-cell reactive clones did not bind at all to the surface of NK-cells. On the other hand, clone 90, which first appeared during the final round of panning on NK-cells as determined by comparing its variable region sequences with those of other zeta-peptide-reactive clones that appeared earlier during the in vitro selection, most likely exhibited low affinity binding to the NK-cell surface without interacting with T-cells. Accordingly, clone 90 did not appear prior to the second round of panning on NK-cells and no binding signal was detectable on either T-cells or NK-cells by flowcytometric analysis of the corresponding Fab-antibody fragments.

### Example 3: Immunization of rats with zeta-peptide-KLH-conjugate and production of anti-zeta-antibodies by the hybridoma technology

At the age of 3 month a Spargue Dawley rat was immunized with the human T-cell line Jurkat (ATCC TIB-152) by intraperitoneal injection of 1 x 10⁷ cells. Three months later the animal was immunized with the zeta-peptide-KLH-conjugate. The conjugate was dissolved in 0.9% NaCl at a concentration of 200 µg/ml. The solution was emulsified 1:2 with complete Freund's adjuvants and 100 µl were injected intraperitonially and subcutaneously. The rat received a booster immunization after 4 weeks in the same way, except that no adjuvants was added. Three days after the boost, the animal was sacrificed and the spleen cells were fused with P3X63Ag8.653 -cells (ATCC CRL-1580) to generate hybridoma cell lines following standard protocols. After PEG-fusion, cells were seeded at 100.000 cells per well in microtiterplates and were grown in 200 µl RPMI 1640 medium supplemented with 10% fetal bovine serum and HAT-additive for selection. After 8 days culture supernatant was completely removed and replaced by fresh medium. After another 4 days, culture supernatant from each well was diluted 1:1 and tested by ELISA (see Example 1). Supematants from those 45 wells exhibiting the strongest reactions with immobilized zeta-peptide-BSA-conjugate were selected for FACS-analysis on CDB⁺-T-lymphocytes and NK-cells that was carried out as described in Example 1 for the murine monoclonal antibodies except that a FITC-labeled anti-rat immunoglobulin antibody (IgG + IgM) (Dianova/Jackson, Hamburg, Cat. No. 112-016-044) was used instead of the FITC-conjugated F(ab')₂-fragment of a rabbit anti-mouse lg antibody. Of the 45 different rat monoclonal antibodies tested, only one clone designated 2-B-5 proved to bind on both CD8⁺-T-lymphocytes and NK-cells. Therefore this clone was analyzed in more detail as described in Examples 4 - 7.

### Example 4: Flowcytometric analysis of the anti-zeta-chain antibody 2-B-5 on CD8⁺-T-cells and NK-cells

In order to test the binding activity of the 2-B-5 antibody on the surface of CD8⁺-T-lymphocytes and NK-cells, mononucleated cells from the peripheral blood of two different healthy donors were isolated by Ficoll-density gradient centrifugation. In each well of a microtiterplate 100.000 mononucleated cells were incubated with undiluted cell culture supernatant of the 2-B-5 hybridoma and with several dilutions thereof, respectively. As negative control culture supernatant of an antibody with the same isotype (rat IgM) but irrelevant specificity was used. After 30 minutes of incubation on ice cells were washed two times with PBS and subsequently stained with two different antibody labeling mixtures. The CD8⁺-T-cells were simultaneously incubated for half an hour on ice with a fluorescein (FITC) conjugated goat-anti-rat Ig (lgG + IgM) antibody (Dianova/Jackson, Hamburg, Cat. No. 112-016-044) diluted 1:100 in PBS, a phycoerythrin (PE) conjugated CD56 antibody (Becton Dickinson, Heidelberg, Cat. No. 347747) diluted 1:25 in PBS and a tricolor conjugated CD3 antibody (Caltac Laboratories, Burlingame, USA, Cod. No. MHCD0306) diluted 1:50 in PBS. To this labeling mixture mouse serum (Sigma Aldrich, St Louis, USA, Cat. No. 054H-8958) was added at a dilution of 1:10 to avoid unspecific binding reactions of the anti-rat antibody to the mouse antibodies.
The NK-cell fraction was incubated with the same goat-anti-rat Ig antibody diluted 1:100 in PBS, a tricolor conjugated CD8 antibody (Caltac Laboratories, Cod. No. MHCD0806) diluted 1:100 in PBS and a phycoerythrin (PE) conjugated CD16 antibody (Becton Dickinson, Heidelberg, Cat. No. 347617) diluted 1:25 in PBS. This mixture was complemented with mouse serum as well. The labeled cells were washed twice in PBS prior to fixation with PBS / 1% paraformaldehyde.

Cells were analyzed by flowcytometry on a FACS-scan (Becton Dickinson, Heidelberg). FACS staining and measuring of the fluorescence intensity were performed as described in Current Protocols in Immunology (Coligan, Kruisbeek, Margulies, Shevach and Strober, Wiley-Interscience, 1992).
Triple color fluorescence analysis was carried out by applying a positive gate for CD8⁺ (Tricolor) and a negative gate for CD16⁺ (PE) cells thus allowing the detection of FITC-mediated fluorescence exclusively attributed to CD8⁺-T-lymohocytes (phenotype: CD8⁺, CD16') without any contaminating signals from CD8⁺-NK-cells. Similarly, triple color fluorescence analysis was carried out by applying a positive gate for CD56⁺- (PE) and a negative gate for CD3⁺-cells (tricolor) thus allowing the detection of FITC-mediated fluorescence exclusively attributed to NK-cells (phenotype: CD56⁺, CD3⁻) without any contaminating signals from CD56⁺-T-lymphocytes.

As shown in Figure 3 the 2-B-5 hybridoma antibody specifically binds to the surface of both T-lymphocytes and NK-cells from different donors.

### Example 5: Confirmation of zeta chain specificity of the 2-B-5 antibody by Sandwich-ELISA

A sandwich ELISA was carried out in order to confirm the zeta-chain-specificity of the monoclonal antibody 2-B-5.
For this purpose, cell lysate from purified CD8⁺-T-lymphocytes, that are known to express the zeta-chain, was prepared and incubated with an immobilized antibody that recognizes the intracellular zeta-chain domain. Zeta-chain molecules from the cell lysate, could than be captured by this antibody and subsequently detected by the 2-B-5 antibody raised against the short extracellular portion of the zeta-chain. Isolation of CD8⁺-lymphcytes was carried out with paramagnetic beads as described in Example 1. The detailed steps were performed according to manufacturers instructions (Dynal, Oslo, Norway). Purified CD8⁺-cells were lysed by the detergent NP-40 (Sigma, Deisenhofen) in presence of the protease inhibitor Phenylmethansulfonylfluoride (PMSF) (Merck, Darmstadt). For detailed buffer formulation see Sambrook, Molecular Cloning; A Laboratory Manual, 2nd Edition, Cold Spring Harbor Laboratory Press, Cold Spring Hobart, NY (1989).

### The sandwich-ELISA was carried out as follows:

A zeta-chain specific antibody (Santa Cruz Biotechnology, Cat-No 1124), which recognizes the amino acids 144-163 at the carboxy-terminus of the zeta chain was coated to wells of a 96 U-bottom plate (Nunc, maxisorb) at a concentration of 5 µg/ml. Coating was performed overnight at 4°C, the following blockade was carried out with 3% BSA in PBS for one hour at room temperature. Subsequently the lysate of CD8⁺⁻cells was added undiluted and in several dilutions and incubated for one hour. As negative control, the wells were incubated with PBS instead of the cell lysate. In the following step the purified monoclonal antibody 2-B-5 was added at a concentration of 1 µg/ml and incubated for one hour. Bound 2-B-5 antibody was detected with a biotinylated mouse-anti-rat IgM antibody (Zymed, San Francisco, CA, USA; Cat-No 03-9840; working concentration 400 ng/ml) followed by an Avidin-peroxidase-conjugate (Dako, Hamburg; Code-No P 03347; working concentration 1 µg/ml). The ELISA was finally developed by addition of ABTS-substrate solution (Boehringer Mannheim, Mannheim, Cat-No. 1682008). The colored precipitate was measured at 405 nm using an ELISA-reader.

The rat IgM antibody 2-B-5 was purified from the hybridoma culture supernatant by ion exchange chromatography using a Bakerbond Abx column (J.T. Baker, Greisheim, Germany) according to the manufacturer's manual.

As shown in Figure 4 the monoclonal antibody 2-B-5 binds to zeta-chain molecules from the T-cell lysate captured by an immobilized antibody recognizing the intracellular zeta-chain domain, with the zeta-specific ELISA-signal strictly depending on the lysate dilution and distinctly ranging above that of the negative control.

### Example 6: Cloning of the variable regions of zeta antibody 2-B-5 and expression of the corresponding Fab-fragment in E. coli

RNA was isolated from 5 x 10⁶ cells of the rat hybridoma cell line 2-B-5 according to the method described by Chomczynski et al. (Anal Biochem, vol. 162, p 156-9 1989). The total RNA was reverse transcribed with the MMLV reverse transcriptase Superscript II (Gibco BRL, Eggenstein) according to standard protocols (Sambrook, Cold Spring Harbour Laboratory Press 1989, second edition). Specific priming of cDNA was carried out with two oligonucleotides designated ratcmuRT (GTGCAGGGCCAGAGAAGGCATC) matching with a short sequence of the constant region of the rat µ-chain and ratckRT (GTAGGTCGCTTGTGGGGAAGTCTC) complementary to a part of the 3'-untranslated region of the light (kappa) chain, with each primer being located approximately 70 basepairs (bp) downstream from the end of the nucleotide sequence encoding the transcript IgM-CH1-heavy chain domain or the constant region of the kappa light chain, respectively. In both cases the nucleotide information was received from the Genebank database (http://www.ncbi.nlm.nih.gov/htbin-post/Entrez/) for the kappa chain: Shepard and Gutman, Accession No. J02574 and for the mu chain: Parker, K.E., Accession No. X68312. The first strand of cDNA was then poly-G tailed using terminal transferase (Pharmacia, Freiburg) according to standard protocol. The tailed cDNA was PCR-amplified using a sense primer containing a poly-C stretch, based on the anchor primer sequence published by Gilliland, L. K. et al., (Tissue Antigens 47, 1-20, 1996) and designated 5'-AncTail (CGTCGATGAGCTCTAGAATTCCCCCCCCCCCCCD) . This anchor primer was combined with an antisense primer, specific for the nucleotide sequence encoding the C-terminus of the kappa light chain constant region or that of the IgM-CH1 heavy chain domain, respectively. The primers were designated 3'ratck (GCGCCGTCTAGAATTAACACTCATTCCTGTTGAA) and 3 ratcmu (ATTGGGACTAGTCTCAACGACAGCTGGAAT). The PCR was carried out as follows: Primary denaturation: 94°C for 4 min.; 30 cycles of amplification: 93°C for 30 sec.; 55°C for 30 sec.; 72°C for 30 sec.; terminal elongation: 72°C for 3 min. Each of these primers contains a restriction enzyme cleavage site (5'-AncTail: *EcoR*I; 3'ratck: *Xba*I; 3'ratcmu: *Spe*I) which allows cloning of the corresponding PCR-fragments into a plasmid vector digested with *EcoR*I/*Xba*I or *Eco*RI/*Spe*I, respectively; for this purpose the bluescript KS+ plasmid vector (Genebank Accession No X52327) was used, since it also allows easy sequence analysis of the resulting inserts by using common sequencing primers. Due to an internal Spel cleavage site within the variable region of the heavy chain (VH) partial digestion followed by cloning of the full length fragment was necessary to obtain the complete sequence information of the VH-domain. Partial digestion was carried out according to standard protocols (Sambrook, Cold Spring Harbour Laboratory Press 1989, second edition). Several clones of heavy and light chain fragments proved to have identical sequences, respectively, and could be identified to encode either functional VL- or VH-regions (see Figure 6 and 7).
The mature N-terminus of both variable chains was identified by comparing their sequences with those found in Genebank database (http://www.ncbi.nlm.nih.gov/htbin-post/Entrez/) and subsequently a second set of PCR-primers was designed to introduce appropriate restriction enzyme cleavage sites in frame with the coding sequences of the 2-B-5 Fab-antibody fragment and with regard to the requirements for subcloning into a bacterial expression vector. The two primers were designated 5'RVHZXhol (CAGGTACAGCTGCTCGAGTCTGGGGC-TGAGCTAG) and 5'RVKZSacI (GTAAATGTGAGCTCCAGATGACACAGTCTCCTG) and used in combination with the 3'ratcmu and 3'ratck primers, respectively. After PCR-amplification and digestion with the appropriate restriction enzyme combinations (*Xho*I/*Spe*I for the heavy chain fragment and *Sac*I/*Xba*I for the kappa light chain), the resulting DNA-fragments were cloned separately into the correspondingly prepared bluescript plasmid vector and sequenced for confirmation (for sequencing results see Fig. 6 and 7).

For the expression of the 2-B-5-Fab-fragment in the periplasma of E. *coli* the corresponding kappa light chain was excised from Bluescript KS+ by using the restriction enzymes *Sac*I/*Xba*I and subcloned into the vector pComb3HHis prepared by digestion with the same enzymes. The resulting plasmid was then digested with the restriction enzymes *Xho*I/*Nhe*I and used as vector for subcloning of the 2-B-5 heavy chain Fd-fragment (VH+CH1); this DNA-fragment was excised from the corresponding Bluescript KS+-clone with the restriction enzymes *Xho*I/*Spe*I.

pComb3HHis was derived from pComb3H and pComb3, respectively (Barbas et al, Proc Natl Acad Sci USA 88, 7978-82 (1991)) by the following modification: The pComb3H vector was cleaved with *Nhe*I and a double stranded oligonucleotide with suitable ends was inserted by ligation. The double stranded oligomer was created through annealing of the two 5'-phosphorylated primers His6s (CTAGCCATCACCATCACCATCACA) and His6as (CTAGTGTGATGGTGATGGTGATGG) (at 94°C, 10 min.; 65°C, 30 min.; 52° C 30 min. and 30°C 10 min.). The primer ends were designed in a way that after fusion with the vector the 3' *Nhe*I restriction site was destroyed whereas the 5' Nhel cleavage site remained intact. Finally, the insert was sequenced to confirm successful cloning.

Periplasma preparation was carried out by osmotic shock and tested by ELISA for Fab-fragments binding to the zeta-peptide-KLH-conjugate. For this purpose single colonies of *E. coli* XL1 Blue transformed with pComb3HHis containing the heavy chain Fd-fragment and the kappa light chain of 2-B-5 were grown in 10 ml Super Broth-medium supplemented with Carbenicilline and 20 mM MgCl₂ and Fab-expression was induced after six hours by adding Isopropyl-β-D-thiogalactosid (IPTG) to a final concentration of 1 mM. The cells were harvested after 20 hours, redissolved in 1 ml PBS. By four rounds of freezing at -70°C and thawing at 37°C, the outer membrane of the bacteria was destroyed and the soluble periplasmatic proteins including the Fab-fragments were released into the supernatant. After elimination of intact cells and cell-debris by centrifugation, the supernatant containing the zeta-Fab-antibody-fragment was collected and used for further examination.

Binding of the periplasmatically expressed Fab-fragment of the cloned monoclonal antibody 2-B-5 to the zeta-peptide-KLH-conjugate was analyzed by the following ELISA: The antigen was immobilized on 96 U well ELISA plates (nunc maxisorb) at a concentration of 200 µg/ml in 50 µl phosphate buffered saline (PBS) per well. Coating was carried out at 4°C for 12 hours followed by single washing step with PBS/0,05% Tween. The ELISA was subsequently blocked for 1 hour with PBS/3% bovine serum albumin (BSA) and washed again once. Then Fab-containing periplasma preparations were added undiluted and in several dilutions and incubated for 2 hours. For detection of Fab-fragments bound to the zeta-peptide-KLH-conjugate a murine anti-His-tag antibody (Dianova, Hamburg, cat. no DIA900) diluted 1:200 was used followed by a peroxidase conjugated polyclonal goat anti-mouse IgG (Fc-gamma specific) (Dianova/Jackson, Hamburg, cat no 115-035-071) antibody diluted 1:5000. The ELISA was finally developed by addition of ABTS-substrate solution (Boehringer Mannheim, Mannheim, Cat-No. 1682008). The turnover of colored substrate was measured by an ELISA-Reader at OD 405 nm; the results are shown in Figure 5. As negative controls, wells were incubated with PBS instead of periplasma preparations.

Specific binding of several clones to the zeta-peptide-KLH-conjugate could be detected with the results of clone 8 and 9 shown in Figure 5. Signal intensities distinctly ranged above those of the negative controls and could be titrated with the sample dilutions. Thus zeta-chain specificity of the cloned 2-B-5-Fab-fragment was confirmed.

### Example 7: Stimulation of T-Lymphocytes and NK-cells by the anti-zeta-chain antibody 2-B-5

The aim of this experiment was to analyze stimulation and proliferation of T-cells, NK-cells and PBMC induced by the anti-zeta-chain antibody 2-B-5. For this purpose a colorimetric immunoassay based on the measurement of Bromodeoxyuridine (BrdU) incorporation during DNA-synthesis was used (Boehringer Mannheim, Mannheim, Cat. No. 1647229).

The first step of this assay was to coat a 96-well flat-bottom microtiterplate with purified of the 2-B-5 antibody in several dilutions (for purification of 2-B-5 see Example 5). Coating was performed overnight at 4°C. After three times of washing with PBS 100.000 CD8⁺-T-lymphocytes, NK-cells and unseparated PBMC, respectively, were added in triplicates to the wells of the microtiterplate, CD8⁺-T-lymphocytes and NK-cells were separated according to the instructions given in Example 2 by using magnetic beads and the primary antibodies anti-CD8 (MT-811) and anti-CD16 (3G8, mouse IgG1, Dianova, Hamburg, Cat. No 0813), respectively.

To control the specificity of 2-B-5 mediated stimulation an antibody of the same isotype (rat IgM) with an irrelevant specificity was used at the same concentrations. The antibody OKT3 (Isotype IgG2a, Ortho., Prod. Code 710320, Johnson + Johnson, New York, USA), which recognizes the human CD3-complex was applied as specific positive control at a coating concentration of 1 µg/ml for the stimulation of T-cells and unseparated PBMC, respectively. A murine IgG2a-antibody of irrelevant specificity was used as isotype control for OKT3. A blank control (wells without cells) and a background control (wells without BrdU) were also included. After an incubation period of three days the BrdU-labeling solution was added for 24 hours. During this labeling period, the pyrimidine analogue BrdU is incorporated in place of thymidine into the DNA of proliferating cells. After removing the culture medium the cells were fixed and the DNA was denatured in one step by adding a denaturation solution. The denaturation of the DNA is necessary to improve the accessibility of the incorporated BrdU for detection by the anti-BrdU-antibody, which is conjugated with peroxidase. This antibody binds to the BrdU incorporated in newly synthesized, cellular DNA.

Bound anti-BrdU-antibody was detected by the subsequent substrate reaction. The reaction product was quantified by an ELISA reader. The turnover of colored substrate as measured by the absorbance values at a wavelength of 450 nm directly correlates with the level of DNA-synthesis and thus with the number of proliferating cells. All steps were performed as described in the manual of the kit manufacturer.

The results of this assay as shown in Figure 8 clearly demonstrate, that the antibody 2-B-5 not only binds to the short extracellular region of the zeta-chain on both T-lymphocytes and NK-cells but that it also induces strong stimulation of both cell types by this interaction.

### Example 8: Flowcytometric analysis of TCR/CD3 complex internalization induced by the binding of the anti-zeta-chain antibody

For many receptors, activation by ligand binding is rapidly followed by receptor internalization. Accordingly, rapid internalization of the TCR-complex on T-cells is typically observed after binding of anti-CD3-antibodies. Thus, internalization of the 2-B-5 antibody after binding to the TCR-complex via its extracellular zeta-chain epitope would confirm the peculiar specificity of the antibody of the invention. (Boyer, C., Auphan, N., Luton, F., Malburet, J. M., Barad, M., Bizozzero, J. P., Reggio, H., and Schmitt-Verhulst, A. M. (1991). T cell receptor/CD3 complex internalization following activation of a cytolytic T cell clone: evidence for a protein kinase C-independent staurosporine-sensitive step. European Journal Of Immunology 21, 1623-34.)

In order to test receptor internalization after binding of the 2-B-5 antibody to the surface of T-cells, a flowcytometric assay was performed at different temperatures, allowing the disappearance of the surface- bound anti-zeta-chain antibody to be observed.

For this purpose mononucleated cells from the peripheral blood of a healthy donor were isolated by Ficoll-density gradient centrifugation. In each well of a microtiterplate 200.000 mononucleated cells were incubated with the anti-zeta-chain antibody 2-B-5 at a concentration of 1 µg/ml, at 4°C or 37°C for either 30 or 60 minutes, thus giving enough time for capping to occur. As positive control a parallel experiment was carried out with a rat-anti-human CD3 antibody (rat IgG2B)(clone 26-II 6-5). The capping process was terminated by washing twice with cold PBS. To label the cell surface bound antibody, the cells were incubated with a fluorescein (FITC) conjugated goat-anti-rat Ig (IgG + IgM) antibody (Dianova/Jackson, Hamburg, Cat. No. 112-016-044) diluted 1:100 in PBS. As negative control, only the secondary antibody was used. The labeled cells were washed twice in PBS prior to fixation with PBS / 0.1 % paraformaldehyde.

Cells were analyzed by flowcytometry on a FACS-scan (Becton Dickinson, Heidelberg). FACS staining and measuring of the fluorescence intensity were performed as described in Current Protocols in Immunology (Coligan, Kruisbeek, Margulies, Shevach and Strober, Wiley-Interscience, 1992).

As the results clearly demonstrated, an apparent shift of fluorescence intensity between the cell samples incubated at 37°C and those incubated at 4°C could be observed after binding of the anti zeta chain antibody 2B5. A similar internalization pattern could be observed after binding of anti-CD3 antibody. In contrast to the receptor internalization at 37°C the samples incubated at 4°C, which is a nonpermissive temperature for capping events, revealed an unaltered fluorescence pattern.

### Example 9: Construction of bispecific antibody based on the anti-zeta chain specificity of the invention

To obtain an anti-zeta scFv-fragment, the corresponding VL- and VH-regions cloned into separate plasmid vectors served as templates for a VL- and VH-specific PCR using the oligonucleotide primer pairs 5'VL2B5BsrGI-EcoRV/3'VL2B5GS15 and 5'VH2B5GS15/3'VH2B5BspEI, respectively. Thereby, overlapping complementary sequences were introduced into the PCR-products, that combine to form the coding sequence of a 15-amino acid (Gly4Ser1)3-linker during the subsequent fusion-PCR.

This amplification step was performed with the primer pair 5'VL2B5BsrGI-EcoRV/3'VH2B5BspEI and the resulting fusion product (or rather anti-zeta-chain scFv-fragment) was cleaved with the restriction enzymes EcoRV and BspEI and thus cloned into a plasmid (described in WO 99/25818) a prepared by digestion with the same restriction enzymes containing a scFv-fragment with binding specificity against the EpCAM antigen as well as a histidine tag at the C-terminus for purification and analytic purposes. Subsequently, the DNA-fragment encoding the anti-zeta-chain/anti-EpCAM bispecific single-chain antibody with the domain arrangement VL_{antiZeta}-VH_{antiZeta}-VH_{antiEpCAM}-VL_{antiEpCAM} was subcloned EcoRI/SalI into the mammalian expression vector pEF-DHFR (Mack, M., Riethmüller, G., and Kufer, P. (1995). A small bispecific antibody construct expressed as a functional single-chain molecule with high tumor cell cytotoxicity. Proc Natl Acad Sci USA 92, 7021-5).
After sequence confirmation (Fig. 10) the resulting plasmid-DNA was transfected into DHFR-deficient CHO-cells by electroporation; selection for stable transfectants, gene amplification and protein production were performed as described (Mack et al). The bispecific antibody was purified via its C-terminal histidine tag by affinity chromatography on a Ni-NTA-column as described (Mack et al).

**List of Primers**

| | |
|---|---|
| 5'VL2B5BsrGl/Eco | 5'-AGG TGT ACA CTC CGA TAT CCA GAT GAC ACA |
| RV | GTC TCC-3' |
| 3'VL 2B5 GS15 | 5'-GGA GCC GCC GCC GCC AGA ACC ACC ACC ACC TTT CAG CTC CAG CTT GGT CCC-3' |
| 5'VH 2B5 GS15 | 5'-GGC GGC GGC GGC TCC GGT GGT GGT GGT TCT CAG GTA CAG CTG CAG CAA TCT GG-3' |
| 3'VH 2B5 BspEI | 5'AAT CCG GAA GAG ACA GTG ACC AGA GTG-3' |

### Example 10: Cytotoxic activity of PBMC and CD8⁺-T-lymphocytes redirected against EpCAM-positive target cells by the bispecific anti-zeta-chain/anti-EpCAM antibody

In this experiment, target cells were labeled with ⁵¹Cr, washed, mixed with effector cells at an effector-to-target ratio of 20:1 and subsequently incubated with different concentrations of the bispecific anti-zeta-chain/anti-EpCAM antibody. The amount of ⁵¹Cr released into the supernatant through target cell killing was quantitated and the rate of specific lysis calculated for each antibody concentration. For this assay human peripheral blood mononuclear cells (PBMCs) or cytotoxic T-lymphocytes were isolated as effector cells from a fresh buffy coat of a healthy donor. PBMCs were separated by ficoll density-gradient centrifugation followed by a subsequent centrifugation step (100 g) to remove thrombocytes. In order to isolate cytotoxic T-Lymphocytes a CD8⁺ Subset column kit (R&D systems, Wiesbaden, Cat-No. HCD8C-1000) was used according to the protocols of the manufacturer. 200 000 unstimulated PBMCs or CD8⁺ T-lymphocytes were added in a volume of 100 µl of RPMI 1640 medium supplemented with 10% FCS to each well of a round-bottomed microtiter plate, respectively. As target cells, Kato III (ATCC HTB-103), an EpCAM positive gastric cancer cell line, labeled for one hour with Chromium-51 (NEN-Life Science, Köln; Cat-No NEZ030S) (with approximately 100µCi) was used; 10.000 target cells in a volume of 100 µl were added to each well of the microtiter plate. The bispecific antibody was added in concentrations from 40 ng/ml to 5 µg/ml in a volume of 50 µl. The microtiter plates were incubated for 16 h at 37° C, 5 % CO₂. At the end of the incubation period 50 µl supernatant were removed from each well and assayed for released ⁵¹Cr in a gamma counter (Wallac, 1480 Wizard 3", Freiburg). Maximal ⁵¹Cr release was determined by the lysis of target cells with a buffer containing Triton-X 100 (1.0% in PBS). The spontaneous ⁵¹Cr release was determined by incubation of target cells without effector cells and bispecific antibody. Incubation of target cells with bispecific antibody did not result in measurable lysis. Specific lysis was calculated as follows: specific release (%) = [(cpm, experimental release) - (cpm, spontaneous release)] / [(cpm, maximal release) - (cpm, spontaneous release)] x 100. All tests were carried out in triplicates. SD within the triplicates was below 6% in all experiments.

The purity of the isolated CD8⁺ effector cells was analyzed by flow cytometry, contaminations with NK-cells were excluded by staining with a PE conjugated anti human CD56 antibody. FACS-analysis was performed as described in example 1. The following antibody conjugates were used: FITC anti human CD3; (Pharmingen Cat-No 30104X) 1:50; PE anti human CD56; (Becton Dickinson Cat-No 347747) 1:20; Tricolor mouse anti human CD8 (Caltac Lab Code NoMHCD0806) 1:100 (Becton Dickinson). The results of the FACS analysis confirmed the high purity of the CD8⁺ cell population (> 99%).

The results (Figure 11) of the Chromium release assay clearly demonstrated the capability of the bispecific anti-zeta-chain/anti-EpCAM antibody to redirect unstimulated PBMCs or CD8⁺ cytotoxic T-lymphocytes against EpCAM positive KATO III cells. The differences between the specific lysis mediated by unseparated PBMC and isolated cytotoxic T-lymphocytes are explained by the cytotoxic contribution of NK-cells.

### Example 11: Cytotoxic activity of NK- cells redirected against EpCAM-positive target cells by the bispecific anti-zeta-chain/anti-EpCAM antibody

This experiment was designed to demonstrate the capability of the bispecific anti-zeta-chain/anti-EpCAM antibody to redirect NK-cells against EpCAM positive target cells. To perform this assay NK-cells were isolated from human peripheral blood mononuclear cells (PBMCs) using an NK-cell isolation kit (Miltenyi Biotec, Bergisch Gladbach; Order No 465-02). The isolation strategy is based on the magnetic depletion of non-NK- cells. T cells, B cells, monocytes, basophils, dendritic cells and platelets are indirectly labeled using a cocktail of hapten-conjugated CD3, CD14, CD19, CD36 and anti-IgE antibodies followed by paramagnetic beads coupled to an anti-hapten monoclonal antibody. Cells associated with magnetic beads were retained by virtue of a magnetic field. The unlabeled NK-cells were washed through the column and remain untouched. 100 000 NK- cells from each of three different healthy donors, were added in a volume of 100 µl RPMI 1640 medium supplemented with 10% FCS to each well of a round-bottomed microtiter plate, respectively. As target, ⁵¹Cr-labeled Kato cells, were added to each well (10 000 target cells per well in a volume of 100 µl each). Bispecific antibody was added in a concentration of 1 µg/ml in a volume of 50 µl. The microtiter plates were incubated for 4h at 37° C, 5 % CO₂. At the end of the incubation period 50 µl supernatant were removed and assayed for released ⁵¹Cr in a gamma counter (Wallac, 1480 Wizard 3", Freiburg). Maximal ⁵¹Cr release was determined by lysis of target cells with a buffer containing a detergent (1.0% Triton-X 100 in PBS). The spontaneous ⁵¹Cr release was determined by incubation of target cells without effector cells and bispecific antibody. Incubation of target cells with bispecific antibody alone did not result in measurable lysis. Specific lysis was calculated as follows: specific release (%)= [(cpm, experimental release) - (cpm, spontaneous release)] / [(cpm, maximal release) - (cpm, spontaneous release)] x 100. All tests were carried out in triplicates. SD within the triplicates was below 6% in all experiments.

The purity of isolated NK-effector cells was analyzed by flow cytometry; contaminations with CD8⁺ cells were excluded by staining with a Tricolor conjugated mouse anti human CD8 antibody. FACS-analysis was performed as described in example 1. The following antibody conjugates were used: FITC anti human CD3; (Pharmingen Cat-No 30104X) 1:50; PE anti human CD56; (Becton Dickinson Cat-No 347747) 1:20; Tricolor mouse anti human CD8 (Caltac Lab Code NoMHCD0806) 1:100 (Becton Dickinson). The results of the FACS analysis confirmed the high purity of the NK- cell population (> 98%).

The results (Figure 12) of the chromium release assay demonstrated in all 3 cases a reproducible capability of redirecting NK- cells against EpCAM positive target cells.

## Claims

1. A nucleic acid molecule comprising a nucleic acid sequence encoding three complementary determining regions (CDRs) of a variable region of an antibody, said antibody specifically interacting with the extracellular domain of the human zeta-chain on the surface of intact cells said antibody being obtainable by immunizing a rat with Jurkat cells and subsequently with a conjugate comprising a carrier molecule and a peptide comprising the 11 N-terminal amino acids of the rat zeta-chain, wherein said 11 N-terminal amino acids consist of the sequence QSFGLLDPKLC wherein said nucleic acid sequence encodes a V_{H} chain; or wherein said nucleic acid sequence encodes a V_{L} chain.

2. The nucleic acid molecule of claim 1 which is a DNA molecule.

3. The nucleic acid molecule of any one of claims 1 or 2, wherein said CDR has one of the following nucleotide sequences:
(a) SEQ ID NO: 1
(b) SEQ ID NO: 3
(c) SEQ ID NO: 5
(d) SEQ ID NO: 7
(e) SEQ ID NO: 9
(f) SEQ ID NO: 11.

4. The nucleic acid moiecuie of claim 1 wherein said V_{H} chain has the nucleotide sequence of SEQ ID No. 13 or encodes the amino acid sequence of SEQ ID No. 14.

5. The nucleic acid molecule of claim 1 wherein said V_{L} chain has the nucleotide sequence of SEQ ID No. 15 or encodes the amino acid sequence of SEQ ID No. 16.

6. The nucleic acid molecule of any one of claims 1 or 2, wherein the CDR encodes one of the amino acid sequences:
(a) SEQ ID No. 2
(b) SEQ ID No. 4
(c) SEQ ID No. 6
(d) SEQ ID No. 8
(e) SEQ ID No. 10
(f) SEQ ID No. 12.

7. A vector comprising the nucleic acid molecule of any one of claims 1 to 6,

8. A host cell transformed or transfected with the vector of claim 7.

9. A method of producing a (poly)peptide encoded by the nucleic acid molecule of any one of claims 1 to 6 comprising culturing the host cell of claim 8 under suitable conditions and isolating said (poly)peptide from the culture.

10. A (poly)peptide encoded by the nucleic acid molecule of any one of claims 1 to 6 or produced by the method of claim 9.

11. An antibody or fragment or derivative thereof comprising at least one (poly)peptide of claim 10.

12. The antibody of claim 11 which is a monoclonal antibody.

13. The antibody of claim 11 which is a bispecific antibody.

14. The antibody of claim 13 wherein the first specificity is for the extracellular domain of the human zeta-chain on the surface of an intact cell and the second specificity is for an optionally different molecule on the surface of a T-lymphocyte, a natural killer cell or a precursor thereof.

15. The antibody of claim 13 wherein the first specificity is for the extracellular domain of the human zeta-chain on the surface of an intact cell and the second specificity is for a different molecule on the surface of a different cell.

16. The antibody of claim 15, wherein said different cell is a cell different from a T-cell, an NK-cell or a precursor thereof.

17. The antibody of claim 15 or 16, wherein said different molecule is a virus encoded antigene, a tumor associated antigen or a surface antigen either on antigen presenting cells (APCs) or on non-APCs:

18. The antibody of claim 17, wherein the APC is a dendritic cell.

19. The derivative of claim 11 which is an scFv chain.

20. The antibody of claim 12 which is an IgM.

21. A bispecific receptor comprising a (poly)peptide of claim 10 and a natural receptor, natural ligand or derivatives thereof interacting with a surface molecule on the same or on another cell.

22. The bispecific receptor of claim 21, wherein said receptors or ligands are CD4, CTLA-4, B7-1, B7-2, LFA-3, ICAM-1, -2, -3 or chemokines like MIP-1α, MIP-1β, RANTES or SDF-1.

23. A pharmaceutical composition comprising the nucleic acid molecule of any of claims 1 to 6, the vector of claim 7, the host cell of claim 8, the (poly)peptide of claim 10, the antibody or fragment or derivative thereof of any one of claims 11 to 20 and/or the bispecific receptor of claim 21 or 22.

24. Use of the antibody of claim 14 for the preparation of a pharmaceutical composition for the treatment or prevention of autoimmune diseases, immune deficiencies, T-cell malignancies, infectious diseases or for the suppression of immune response.

25. The use of claim 24, wherein said suppression of immune response is to be in order to avoid graft rejection after organ transplantation.

26. Use of the antibody of claim 15 for the preparation of a pharmaceutical composition of the treatment or prevention of malignancies, viral infections, or other infectious diseases.

27. Use of the (poly)peptide of claim 10 or the antibody or fragment or derivative thereof of any one of claims 11 to 20 or the bispecific receptor of claim 21 or 22 for the preparation of a pharmaceutical composition for the enhancement or suppression of NK-cell dependent immunity or for the treatment of NK-cell derived malignancies.

28. An in vitro method for the determination of zeta-chain or eta-chain expression on NK-cells, T-lymphocytes or precursors thereof comprising
(a) contacting the (poly)peptide of claim 10 or the antibody or fragment or derivative thereof of any one claims 11 to 20 with said NK-cells, T-lymphocytes or precursors thereof; and
(b) assessing the amount of bound (poly)peptide, antibody or derivative.

29. A kit comprising the nucleic acid molecule of any of claims 1 to 6, the vector of claim 7, the host cell of claim 8, the (poly)peptide of claim 10, the antibody or fragment or derivative thereof of any one of claims 11 to 20 and/or the bispecific receptor of claim 21 or 23.

30. A non-human transgenic animal comprising in its germline at least one copy of the nucleic acid molecule of any of claims 1 to 6 or the vector of claim 7.

## Patentansprüche

1. Nucleinsäuremolekül, umfassend eine Nucleinsäuresequenz, die drei Komplementaritäts-bestimmende Regionen (CDRs) einer variablen Region eines Antikörpers codiert, wobei der Antikörper mit der extrazellulären Domäne einer menschlichen Zeta-Kette auf der Oberfläche von intakten Zellen spezifisch wechselwirkt, wobei der Antikörper erhältlich ist durch Immunisieren einer Ratte mit Jurkat-Zellen und anschließend mit einem Konjugat, umfassend ein Trägermolekül und ein Peptid, das die 11 N-terminalen Aminosäuren der Ratten Zeta-Kette umfasst, wobei diese 11 N-terminalen Aminosäuren aus der Sequenz QSFGLLDPKLC bestehen, wobei die Nucleinsäuresequenz eine V_{H-}Kette codiert; oder wobei die Nulceinsäuresequenz eine V_{L}-Kette codiert.

2. Nucleinsäuremolekül nach Anspruch 1, das ein DNA-Molekül ist.

3. Nucleinsäuremolekül nach Anspruch 1 oder 2, wobei die CDR eine der folgenden Nucleotidsequenzen besitzt:
(a) SEQ ID NO:1
(b) SEQ ID NO:3
(c) SEQ ID NO:5
(d) SEQ ID NO:7
(e) SEQ ID NO:9
(f) SEQ ID NO:11.

4. Nucleinsäuremolekül nach Anspruch 1, wobei die V_{H}-Kette die Nucleotidsequenz von SEQ ID NR:13 besitzt oder die Aminosäuresequenz von SEQ ID NO:14 codiert.

5. Nucleinsäuremolekül nach Anspruch 1, wobei die V_{L}-Kette die Nucleotidsequenz von SEQ ID NO:15 besitzt oder die Aminosäuresequenz von SEQ ID NO:16 codiert.

6. Nucleinsäuremolekül nach einem der Ansprüche 1 oder 2, wobei die CDR eine der folgenden Aminosäuresequenzen codiert:
(a) SEQ ID NO:2
(b) SEQ ID NO:4
(c) SEQ ID NO:6
(d) SEQ ID NO:8
(e) SEQ ID NO:10
(f) SEQ ID NO:12.

7. Vektor, umfassend das Nucleinsäuremolekül nach einem der Ansprüche 1 bis 6.

8. Wirtszelle, transformiert oder transfiziert mit einem Vektor nach Anspruch 7.

9. Verfahren zur Herstellung eines (Poly)peptids, das durch ein Nucleinsäuremolekül nach einem der Ansprüche 1 bis 6 codiert wird, umfassend das Züchten der Wirtszelle nach Anspruch 8 unter geeigneten Bedingungen und das Isolieren des (Poly)peptids aus der Kultur.

10. (Poly)peptid, das durch ein Nucleinsäuremolekül nach einem der Ansprüche 1 bis 6 codiert wird oder nach einem Verfahren nach Anspruch 9 hergestellt wird.

11. Antikörper oder Fragment oder Derivat davon, umfassend mindestens ein (Poly)peptid nach Anspruch 10.

12. Antikörper nach Anspruch 11, der ein monoclonaler Antikörper ist.

13. Antikörper nach Anspruch 11, der ein bispezifischer Antikörper ist.

14. Antikörper nach Anspruch 13, wobei die erste Spezifität für die extrazelluläre Domäne der menschlichen Zeta-Kette auf der Oberfläche einer intakten Zelle ist und die zweite Spezifität für ein fakultativ unterschiedliches Molekül auf der Oberfläche eines T-Lymphozyten, einer natürlichen Killerzelle oder eines Vorläufers davon ist.

15. Antikörper nach Anspruch 13, wobei die erste Spezifität für die extrazelluläre Domäne der menschlichen Zeta-Kette auf der Oberfläche einer intakten Zelle ist und die zweite Spezifität für ein unterschiedliches Molekül auf der Oberfläche einer unterschiedlichen Zelle ist.

16. Antikörper nach Anspruch 15, wobei die unterschiedliche Zelle eine Zelle ist, die sich von einer T-Zelle, einer NK-Zelle oder einem Vorläufer davon unterscheidet.

17. Antikörper nach Anspruch 15 oder 16, wobei das unterschiedliche Molekül ein Virus-codiertes Antigen, ein tumorassoziiertes Antigen oder ein Oberflächenantigen entweder auf Antigen-präsentierenden Zellen (APCs) oder auf Nicht-APCs ist.

18. Antikörper nach Anspruch 17, wobei die APC eine dendritische Zelle ist.

19. Derivat nach Anspruch 11, das eine scFv-Kette ist.

20. Antikörper nach Anspruch 12, der ein IgM ist.

21. Bispezifischer Rezeptor, umfassend ein (Poly)peptid nach Anspruch 10 und einen natürlichen Rezeptor, einen natürlichen Liganden oder Derivate davon, die mit einem Oberflächenmolekül auf derselben oder einer anderen Zelle wechselwirken.

22. Bispezifischer Rezeptor nach Anspruch 21, wobei die Rezeptoren oder Liganden CD4, CTLA-4, B7-1, B7-2, LFA-3, ICAM-1, -2, -3 oder Chemokine wie MIP-1α, MIP-1β, RANTES oder SDF-1 sind.

23. Arzneimittel, umfassend das Nucleinsäuremolekül nach einem der Ansprüche 1 bis 6, den Vektor nach Anspruch 7, die Wirtszelle nach Anspruch 8, das (Poly)peptid nach Anspruch 10, den Antikörper oder das Fragment oder Derivat davon nach einem der Ansprüche 11 bis 20 und/oder den bispezifischen Rezeptor nach Anspruch 21 oder 22.

24. Verwendung des Antikörpers nach Anspruch 14 für die Herstellung eines Arzneimittels zur Behandlung von oder zur Vorbeugung vor AutoimmunErkrankungen, Immunschwächen, T-Zell-Malignitäten, Infektionserkrankungen oder zur Unterdrückung einer Immunantwort.

25. Verwendung nach Anspruch 24, wobei die Unterdrückung einer Immunantwort dazu dient, eine Organabstoßung nach einer Organtransplantation zu verhindern.

26. Verwendung des Antikörpers nach Anspruch 15 für die Herstellung eines Arzneimittels zur Behandlung von oder zur Vorbeugung vor Malignitäten, viralen Infektionen oder anderen Infektionserkrankungen.

27. Verwendung des (Poly)peptids nach Anspruch 10 oder des Antikörpers oder Fragments oder Derivats davon nach einem der Ansprüche 11 bis 20 oder des bispezifischen Rezeptors nach Anspruch 21 oder 22 für die Herstellung eines Arzneimittels zur Verstärkung oder Unterdrückung einer NK-Zell-abhängigen Immunität oder zur Behandlung von NK-Zell-abgeleiteten Malignitäten.

28. In vitro Verfahren zur Bestimmung der Zeta-Ketten- oder Eta-Ketten-Expression auf NK-Zellen, T-Lymphozyten oder Vorläufern davon, umfassend:
(a) Inkontaktbringen des (Poly)peptids nach Anspruch 10 oder des Antikörpers oder Fragments oder Derivats davon nach einem der Ansprüche 11 bis 20 mit den NK-Zellen, T-Lymphozyten oder Vorläufern davon; und
(b) Beurteilen der Menge des gebundenen (Poly)peptids, Antikörpers oder Derivats.

29. Kit, umfassend das Nucleinsäuremolekül nach einem der Ansprüche 1 bis 6, den Vektor nach Anspruch 7, die Wirtszelle nach Anspruch 8, das (Poly)peptid nach Anspruch 10, den Antikörper oder das Fragment oder Derivat davon nach einem der Ansprüche 11 bis 20 und/oder den bispezifischen Rezeptor nach Anspruch 21 oder 22.

30. Nicht-menschliches transgenes Tier, umfassend in dessen Keimbahn mindestens eine Kopie des Nucleinsäuremoleküls nach einem der Ansprüche 1 bis 6 oder des Vektors nach Anspruch 7.

## Revendications

1. Molécule d'acide nucléique comprenant une séquence d'acide nucléique codant trois régions de détermination de complémentarité (CDR) d'une région variable d'un anticorps, ledit anticorps interagissant spécifiquennent avec le domaine extracellulaire de la chaîne zêta humaine à la surface de cellules intactes, ledit anticorps pouvant être obtenu en immunisant un rat avec des cellules jurkat puis avec un conjugué comprenant une molécule porteuse et un peptide comprenant les 11 acides aminés N-terminaux de la chaîne zêta de rat, où lesdits 11 acides aminés N-terminaux consistent en la séquence QSFGLLDPKLC, où ladite séquence d'acide nucléique code pour une chaîne V_{H}; ou où ladite séquence d'acide nucléique code pour une chaîne V_{L}.

2. Molécule d'acide nucléique selon la revendication 1 qui est une molécule d'ADN.

3. Molécule d'acide nucléique selon l'une quelconque de la revendication 1 ou de la revendication 2, dans laquelle ladite CDR a l'une des séquences nucléotidiques suivantes :
(a) SEQ ID N° 1
(b) SEQ ID N° 3
(c) SEQ ID N° 5
(d) SEQ ID N° 7
(e) SEQ ID N° 9
(f) SEQ ID N° 11.

4. Molécule d'acide nucléique selon la revendication 1 dans laquelle ladite chaîne V_{H} a la séquence nucléotidique de SEQ ID N° 13 ou code la séquence d'acides aminés de SEQ ID N° 14.

5. Molécule d'acide nucléique selon la revendication 1 dans laquelle ladite chaîne V_{L} a la séquence nucléotidique de SEQ ID N° 15 ou code la séquence d'acides aminés de SEQ ID N° 16.

6. Molécule d'acide nucléique selon l'une quelconque de la revendication 1 ou de la revendication 2, dans laquelle la CDR code l'une des séquences d'acides aminés suivantes :
(a) SEQ ID N° 2
(b) SEQ ID N° 4
(c) SEQ ID N° 6
(d) SEQ ID N° 8
(e) SEQ ID N° 10
(f) SEQ ID N° 12.

7. Vecteur comprenant la molécule d'acide nucléique selon l'une quelconque des revendications 1 à 6.

8. Cellule hôte transformée ou transfectée avec le vecteur selon la revendication 7.

9. Procédé pour produire un (poly)peptide codé par la molécule d'acide nucléique selon l'une quelconque des revendications 1 à 6 comprenant la mise en culture de la cellule hôte de la revendication 8 dans des conditions adaptées et l'isolement dudit (poly)peptide dans la culture.

10. (Poly)peptide codé par la molécule d'acide nucléique selon l'une quelconque des revendications 1 à 6 ou produit par le procédé selon la revendication 9.

11. Anticorps ou fragment ou dérivé de celui-ci comprenant au moins un (poly)peptide selon la revendication 10.

12. Anticorps selon la revendication 11 qui est un anticorps monoclonal.

13. Anticorps selon la revendication 11 qui est un anticorps bispécifique.

14. Anticorps selon la revendication 13 dans lequel la première spécificité est pour le domaine extracellulaire de la chaîne zêta humaine à la surface d'une cellule intacte et la seconde spécificité est pour une molécule facultativement différente à la surface d'un lymphocyte T, d'une cellule natural killer ou d'un précurseur de ceux-ci.

15. Anticorps selon la revendication 13 dans lequel la première spécificité est pour le domaine extracellulaire de la chaîne zêta humaine à la surface d'une cellule intacte et la seconde spécificité est pour une molécule différente à la surface d'une cellule différente.

16. Anticorps selon la revendication 15, dans lequel ladite cellule différente est une cellule différente d'un lymphocyte T, d'une cellule NK ou d'un précurseur de ceux-ci.

17. Anticorps selon la revendication 15 ou la revendication 16, dans lequel ladite molécule différente est un antigène codé par un virus, un antigène tumoral-associé ou un antigène de surface soit sur des cellules présentatrices de l'antigène (CFA) soit sur des cellules non CPA.

18. Anticorps selon la revendication 17 dans lequel la CPA est une cellule dendritique.

19. Dérivé selon la revendication 11 qui est une chaîne scFv.

20. Anticorps selon la revendication 12 qui est une IgM.

21. Récepteur bispécifique comprenant un (poly)peptide selon la revendication 10 et un récepteur naturel, un ligand naturel ou des dérivés de ceux-ci interagissant avec une molécule de surface sur la même cellule ou sur une autre cellule.

22. Récepteur bispécifique selon la revendication 21, dans lequel lesdits récepteurs ou ligands sont CD4, CTLA-4, B7-1, B7-2, LFA-3, ICAM-1, -2, -3 ou des chimiokines comme MIP-1α, MIP-1β, RANTES ou SDF-1.

23. Composition pharmaceutique comprenant la molécule d'acide nucléique selon l'une quelconque des revendications 1 à 6, le vecteur selon la revendication 7, la cellule hôte selon la revendication 8, le (poly)peptide selon la revendication 10, l'anticorps ou le fragment ou dérivé de celui-ci selon l'une quelconque des revendications 11 à 20 et/ou le récepteur bispécifique selon la revendication 21 ou la revendication 22.

24. Utilisation de l'anticorps selon la revendication 14 pour la préparation d'une composition pharmaceutique pour le traitement ou la prévention de maladies auto-immunes, de déficiences immunitaires, d'affections malignes des lymphocytes T, de maladies infectieuses ou pour l'inhibition de la réponse immunitaire.

25. Utilisation selon la revendication 24, dans laquelle ladite inhibition de la réponse immunitaire vise à éviter le rejet de greffon après transplantation d'organe.

26. Utilisation de l'anticorps selon la revendication 15 pour la préparation d'une composition pharmaceutique pour le traitement ou la prévention d'affections malignes, d'infections virales ou d'autres maladies infectieuses.

27. Utilisation du (poly)peptide selon la revendication 10 ou de l'anticorps ou fragment ou dérivé de celui-ci selon l'une quelconque des revendications 11 à 20 ou du récepteur bispécifique selon la revendication 21 ou la revendication 22 pour la préparation d'une composition pharmaceutique pour le renforcement ou l'inhibition de l'immunité dépendante des cellules NK ou pour le traitement d'affections malignes dérivées des cellules NK.

28. Procédé *in vitro* pour la détermination de l'expression de la chaîne zêta ou de 1a chaîne êta sur des cellules NK, des lymphocytes T ou leurs précurseurs comprenant les étapes consistant à :
(a) mettre en contact le (poly)peptide selon la revendication 10 ou l'anticorps ou fragment ou dérivé de celui-ci selon l'une quelconque des revendications 11 à 20 avec lesdites cellules NK, lesdits lymphocytes T ou les précurseurs de ceux-ci ; et
(b) évaluer la quantité de (poly)peptide, d'anticorps ou de dérivé liés.

29. Kit comprenant la molécule d'acide nucléique selon l'une quelconque des revendications 1 à 6, le vecteur selon la revendication 7, la cellule hôte selon la revendication 8, le (poly)peptide selon la revendication 10, l'anticorps ou le fragment ou dérivé de celui-ci selon l'une quelconque des revendications 11 à 20 et/ou le récepteur bispécifique selon la revendication 21 ou 23.

30. Animal transgénique non humain comprenant dans sa lignée germinale au moins un exemplaire de la molécule d'acide nucléique selon l'une quelconque des revendications 1 à 6 ou le vecteur selon la revendication 7.
